# EUROPEAN PATENT APPLICATION

(11) **EP 2 399 904 A1**
(43) Date of publication of application: **28.12.2011**
(21) Application number: 10450092.1
(22) Date of filing: 26.05.2010
(51) Int. Cl.: C07C 323/52, A61K 31/215, A61P 17/00, A61P 31/00

(54) **Process for the preparation of pleuromutilins**

(71) Applicant: Nabriva Therapeutics AG, 1112 Wien (AT)
(72) Inventor: Mang, Rosemarie, 1220 Wien (AT); Heilmayer, Werner, Dr., 7034 Zillingtal (AT); Spence, Lee, 1050 Wien (AT)
(74) Representative: Schwarz, Albin

(57) **Abstract**

A process for the preparation of a compound of formula 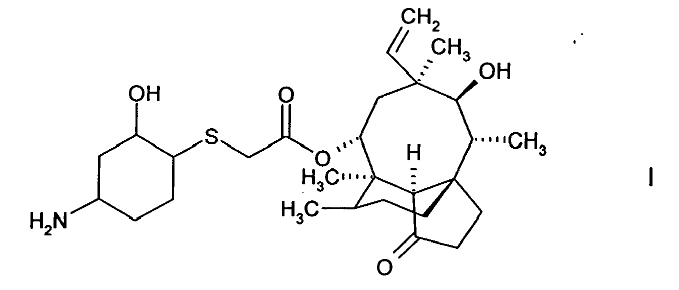 in the form of a single stereoisomer, comprising deprotecting the amine group in an N-protected amino-hydroxy-cyclohexylsulfanyl-acetyl-mutilin of formula 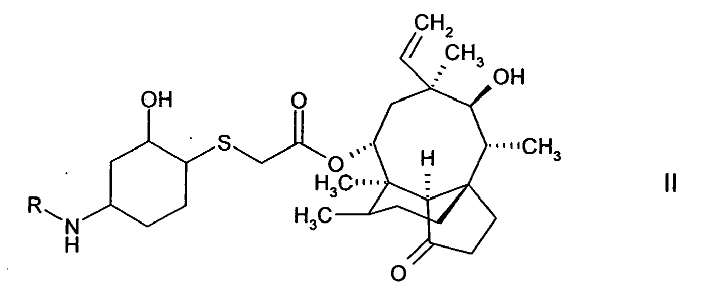 wherein R is an amine protecting group in the form of a single stereoisomer,
and isolating a compound of formula I in the form of a single stereoisomer obtained from the reaction mixture; compounds obtainable by such processes, e.g. a compound of formula I in a crystalline form, or salts of a compound of formula I in crystalline form, and processes for the preparation of intermediates for the production of a compound of formula I.

## Description

The present invention relates to a new process for the preparation of 14-O-{[(4-Amino-2-hydroxy-cyclohexyl)-sulfanyl]-acetyl}-mutilins.

Pleuromutilin, a compound of formula 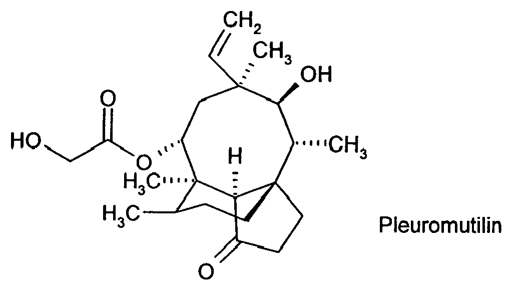 is a naturally occurring antibiotic, e.g. produced by the basidomycetes Pleurotus mutilus and P. passeckerianus, see e.g. The Merck Index, 12th edition, item 7694.

A number of further pleuromutilins having the principle ring structure of pleuromutilin and being substituted at the primary hydroxy group have been developed, e.g. as antimicrobials. Due to their pronounced antimicrobial activity, a group of pleuromutilin derivatives, amino-hydroxy-substituted cyclohexylsulfanylacetylmutilins, as disclosed in WO 2008/113089, have been found to be of particular interest. As described in WO2008/1108 14-0-{[(4-Amino-2-hydroxy-cyclohexyl)-sulfanyl]-acetyl}-mutilins are particularly useful compounds because they demonstrate activity against Gram-positive and Gram-negative pathogens e.g. associated with respiratory tract and skin and skin structure infections. For the production of substantially pure isomers/diasteromers of this group of compounds, there is a need for a production process which is convenient for use on an industrial scale and which also avoids the use of costly starting materials, environmentally hazardous reagents and solvents or time consuming and laborious purification steps. The production process described in WO 2008/113089 involves chromatographic purification of the compounds prepared according to individual synthesis steps and the final diastereomers are separated by chiral HPLC chromatography which cannot be used on industrial scale.

Surprisingly, production processes for stereochemically pure amino-hydroxy-substituted cyclohexylsulfanylacetylmutilins have now been found wherein chromatographic seperation steps, such as chiral HPLC chromatography may be avoided.

In one aspect the present invention provides a process for the preparation of a compound of formula 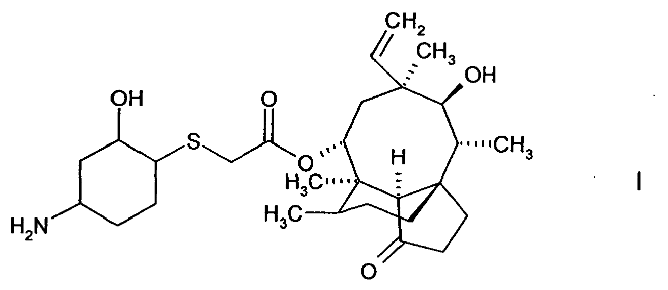 in the form of a single stereoisomer, e.g. including a compound of formula 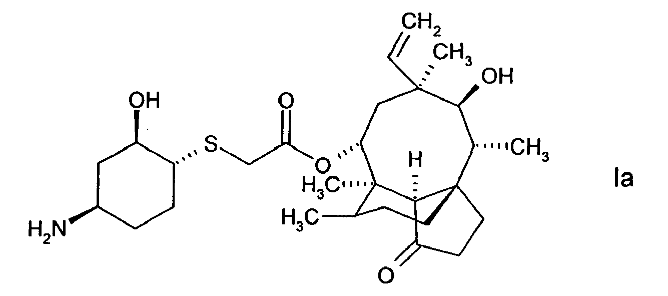 and a compound of formula 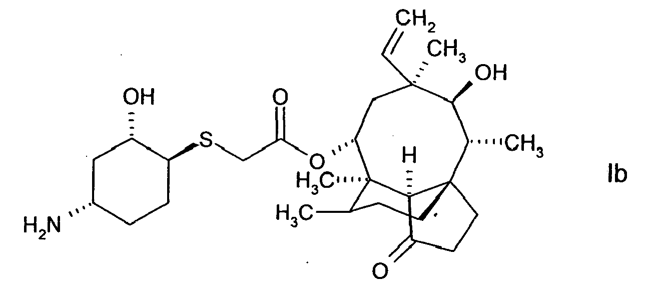 comprising deprotecting the amine group in an N-protected amino-hydroxy-cyclohexylsulfanyl-acetyl-mutilin of formula 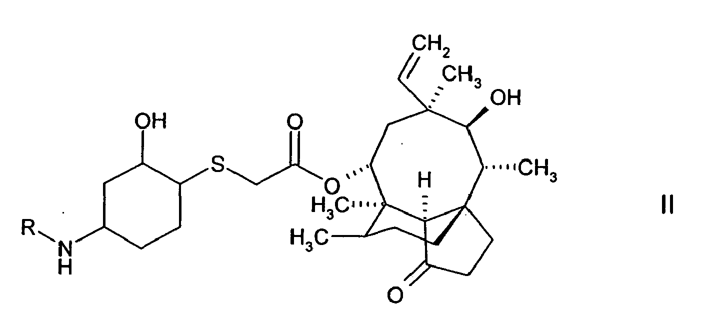 in the form of a single stereoisomer, e.g. including a compound of formula 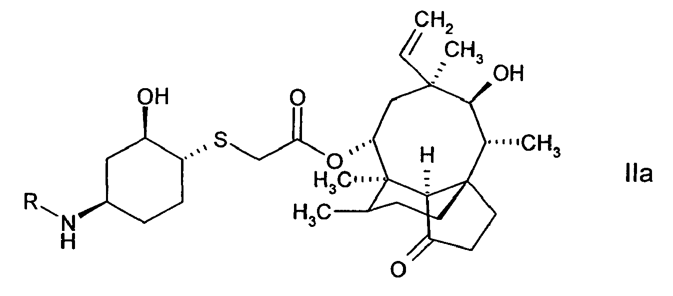 and a compound of formula 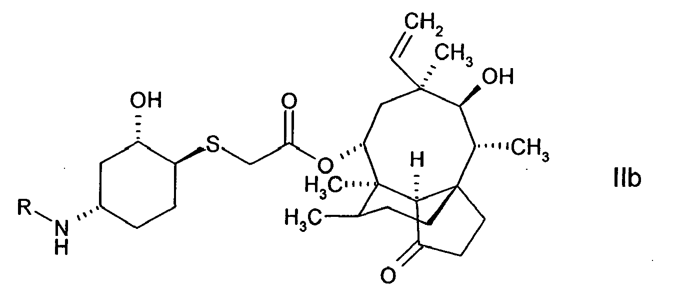 wherein R is an amine protecting group;
and isolating a compound of formula I in the form of a single stereoisomer, e.g. a compound of formula Ia or Ib, obtained from the reaction mixture.

Compounds of formula IIa and IIb are new and also form part of the present invention.

In another aspect the present invention provides a compound of formula IIa or IIb.

In a compound of formula Ia, or IIa, respectively, the carbon atoms of the cyclohexyl ring to which the hydroxy group, the amine group and the sulfanyl-acetyl-mutilin group are attached are all in the R configuration and thus a compound of formula Ia, or IIa represents an optionally amine protected 14-O-{[(1*R*,2*R*,4*R*)-4-Amino-2-hydroxy-cyclohexylsulfanyl]-acetyl}-mutilin; and in a compound of formula Ib, or IIb, respectively, the carbon atoms of the cyclohexyl ring to which the hydroxy group, the amine group and the sulfanyl-acetyl-mutilin group are attached are all in the S configuration and thus a compound of formula Ib, or IIb, respectively, represents an optionally amino protected 14-O-{[(1*S*,2*S*,4*S*)-4-Amino-2-hydroxy-cyclohexylsulfanyl]-acetyl}-mutilin.

An amine protecting group includes protecting groups known to a skilled person and which are removable under acidic, basic, hydrogenating, oxidative or reductive methods, e.g. by hydrogenolysis, treatment with an acid, a base, a hydride, a sulfide. Appropriate amine protecting groups e.g. are described in T. W. Greene, P. G. M. Wuts, Protective Groups in Organic Synthesis, Wiley-Interscience, New York, 1999, particularly p. 503-607, 736-747.

Amine protecting groups which may be conveniently used in a process according to the present invention include for example
- benzyloxycarbonyl (Cbz), removable e.g. by hydrogenolysis,
- *p*-methoxybenzyl carbonyl (Moz or MeOZ), removable e.g. by hydrogenolysis,
- *tert*-butyloxycarbonyl (BOC), removable e.g. by treatment with a strong acid, such as HCl, H₃PO₄ or CF₃COOH,
- 9-fluorenylmethyloxycarbonyl (FMOC), removable e.g. by treatment with a base, such as piperidine,
- benzyl (Bn), removable e.g. by hydrogenolysis;
- *p*-methoxybenzyl (PMB), removable e.g. by hydrogenolysis;
- 3,4-dimethoxybenzyl (DMPM), removable e.g. by hydrogenolysis;
- *p*-methoxyphenyl (PMP), removable e.g. by treatment with ammonium cerium(IV) nitrate (CAN),
- tosyl (Tos), removable e.g. by treatment with concentrated acid, such as HBr, H₂SO₄, or by treatment with strong reducing agents, such as sodium in liquid ammonia, sodium naphthalene,
- groups which form with the amine sulfonamides other than Tos-amides, e.g. including 2-nitrobenzenesulfonamide (nosyl) or o-nitrophenylsulfenyl (Nps), removable e.g.by treatment with samarium iodide, tributyltin hydride,
- benzylidene, removable e.g. by treatment with trifluoromethanesulfonic acid, trifluoroacetic acid, dimethyl sulfide;
- triphenylmethyl (trityl, Tr), dimethoxytrityl (DMT), e.g. removable by treatment with an acid, such as trifluoroacetic acid;
preferably BOC.

An acid, e.g. an inorganic, such as a mineral acid, or an organic acid may be used to effect deprotection of a compound of formula II in the form of a single stereoisomer to obtain a compound of formula I in the form of a single stereoisomer. In a preferred embodiment an organic acid, such as trifluoroacetic acid (TFA), or a mineral acid, such as ortho phosphoric acid is used.

It has been found that during the amine deprotection reaction the stereochemistry of carbon atoms of the cyclohexyl moiety wherein the thio, hydroxy and amino group, respectively, are attached is retained and remains the same in a compound of formula I, as in the amine protected compound of formula II used as a starting material.

Surprisingly it has been found that a compound of formula I in a single stereoisomeric form, such as a compound of formula Ia, may be crystallized after isolation, or even directly in the reaction mixture obtained after deprotection of the amine group. For example a solution of a compound of formula I in a single stereoisomeric form obtained in organic solvent, preferably polar organic solvent, such as CH₂Cl₂, is treated with an antisolvent, such as diisopropylether (DIPE); or an isolated crude material of a compound of formula I in a single stereoismeric form, e.g. a compound of formula Ia, is taken up in an organic solvent, such as an ether, e.g. tetrahydrofuran (THF). A compound of formula I in a single stereoisomeric form, e.g. of formula Ia, may crystallize and may be isolated in crystalline form.

Crystallization simplifies isolation and handling of the isolated materials and provides excellent opportunities for further purification of the product.

The crystalline compound obtained may be subjected to further purification, such as recrystallization e.g. out of an organic solvent, preferably an alcohol, such as n-butanol.

(Re)crystallization may be repeated if desired and may result in high recovery yields and excellent purification.

In the following TABLE 1 the purification potential of the crystalline compound of formula Ia is indicated.

**TABLE 1**

| **la** | **HPLC purity (% area) of la** | **Impurity RRT 1.32 (% area)** |
|---|---|---|
| Reaction completion | 87.3 | 4.2 |
| After DCM/DIPE crystallisation | 95.0 | 2.2 |
| After 1^{st} n-BuOH crystallisation | 99.0 | 0.6 |
| After 2^{nd} n-BuOH crystallisation ³ | 99.7 | <0.15 |

| | | |
|---|---|---|
| RRT = relative retention time in the HPLC with respect to Ia | | |

Crystallization may be enhanced and accelerated by the use of seed crystals. The crystallization and recrystallization process of a compound Ia delivers the desired purity and is highly useful on an industrial scale. The cumbersome and expensive purification by chromatography is circumvented and makes the process industrially applicable.

In a further aspect the present invention provides a compound of formula I, such as of formula Ia, in single stereoisomeric form in crystalline form.

Surprisingly, two different crystalline forms of a compound of formula Ia have been isolated and characterized by X-ray powder diffraction.

In another aspect the present invention provides 14-*O*-{[(1*R*,2*R*,4*R*)-4-amino-2-hydroxycyclohexylsulfanyl]-acetyl}-mutilin Form 1, in crystalline form, e.g. which is characterized by an X-ray powder diffraction pattern with peaks 2-theta at (degrees, ± 0.2, *inter alia*):
10.6, 11.1, 12.0, 14.3, 15.1, 16.1, 21.1; such as:
   10.6, 11.1, 12.0, 14.3, 15.1, 16.1, 18.2, 19.2, 20.7, 21.1, 21.3, 21.8, 22.6, 23.5, 24.7, 28.2, 30.2.

In another aspect the present invention provides crystalline 14-*O*-{[(1*R*,2*R*,4*R*)-4-amino-2-hydroxy-cyclohexylsulfanyl]-acetyl}-mutilin, Form 2, e.g. which is characterized by an X-ray powder diffraction pattern with peaks 2-theta at (degrees, ± 0.2, *inter alia*):
9.8, 11.1, 13.1, 14.1, 17.6, 19.7, 22.2; such as
   9.6, 9.8, 11.1, 13.1, 14.1, 16.0, 17.6, 19.7, 22.2; 22.7, 23.0.

A compound of formula I is preferably isolated in the form of a salt, such as a pharmaceutically acceptable salts. Such salts are preferably ammonium salts, namely acid addition salts of the amino group attached to the cyclohexyl ring, and include acetates, lactates, e.g. L-lactates, or maleates.

In another aspect the present invention provides a compound of formula I in the form of a single stereoisomer and in the form of a salt, e.g. crystalline salt, wherein a compound of formula I in the form of a single stereoisomer preferably is a compound of formula Ia; the salt preferably is an acetate, lactate, e.g. L-lactate or maleate.

In a still further aspect the present invention provides a pharmaceutical composition comprising crystalline 14-O-{[(4-amino-2-hydroxycyclohexyl)sulfanyl]acetyl}-mutilin, or comprising an, optionally, e.g. preferably crystalline, acetate, lactate, or maleate of 14-O-{[(4-amino-2-hydroxycyclohexyl)sulfanyl]acetyl}-mutilin.

Compounds of formula I in free form and in single stereoisomeric form thus obtained may be converted into a salt by addition of an acid. The free base of a compound of formula I, preferably of formula Ia, is dissolved or suspended, or is obtained in dissolved or suspended form, in organic solvent, preferably apolar solvent, such as methyl acetate, ethyl acetate, propyl acetate, e.g. isopropyl acetate, and to the mixture obtained an acid, e.g. an organic acid or an inorganic (mineral) acid, e.g. acetic acid, lactic acid, e.g. L-lactic acid, maleic acid, is added. A compound of formula I, such as of formula Ia, in the form of a salt, e.g. crystalline salt, may be obtained and may be isolated.

It has been found that in the salt formation step the stereochemistry of the carbon atoms of the cyclohexyl ring to which a thio, hydroxy or an amino group is attached, is retained and remains the same as in a compound of formula I in free form.

Isolation of pharmaceutically active compounds as described in WO2008/113089, example I and Ia, respectively, but according to the present invention in crystalline salt form, such as a compound of formula I, e.g. formula Ia in crystalline salt form, is highly advantageous. The improved purity and stability of the crystalline salt is very important and useful for the preparation of pharmaceutical compositions, intended for veterinary and human use. The crystalline salts of a compound of formula I, e.g. Ia, show improved stability over the corresponding amorphous forms and are isolated with chemical and chiral purity of ≥ 90%, e.g. even ≥ 95%.

The antimicrobial, e.g. antibacterial activity of 14-O-{[(4-Amino-2-hydroxy-cyclohexyl)-sulfanyl]-acetyl}-mutilins was outlined in WO2008/113089 For example, the compounds of the present invention e.g. compound of formula Ia or formula Ib show antimicrobial, e.g. antibacterial, activity against Gram-positive bacteria, such as coagulase positive Staphylococci, e.g. Staphylococcus aureus and Streptococci, e.g. Streptococcus pneumoniae, e.g. exhibiting MICs < 0.4 µg/ml against Staphylococcus aureus ATCC49951 and Streptococcus pneumoniae ATCC49619. The minimum inhibitory concentration (MIC) was determined in accordance with CLSI recommendations.

In a further aspect the present invention provides crystalline 14-*O*-{[(1*R*,2*R*,4*R*)-4-amino-2-hydroxy-cyclohexylsulfanyl]-acetyl}-mutilin in the form of an acetate, Form A, e.g. which is characterized by an X-ray powder diffraction pattern with peaks 2-theta at (degrees, ± 0.2, *inter alia*):
7.0, 7.7, 11.6, 12.1, 12.6, 13.5, 13.7, 15.4, 15.7, 16.9, 17.3, 19.0, 19.9, 21.1, 23.4, 24.2, 24.4; such as
   7.0, 7.7, 11.6, 12.1, 12.6, 13.5, 13.7, 14.1, 15.4, 15.7, 16.5, 16.9, 17.3, 19.0, 19.6, 19.9, 20.1, 21.1, 22.2, 22.5, 23.4, 24.2, 24.4, 26.7, 29.1, 29.6, 31.0.

In a further aspect the present invention provides crystalline 14-*O*-{[(1*R*,2*R*,4*R*)-4-amino-2-hydroxy-cyclohexylsulfanyl]-acetyl}-mutilin in the form of an acetate, Form B, e.g. which is characterized by an X-ray powder diffraction pattern with peaks 2-theta at (degrees, ± 0.2, *inter alia*):
10.3, 10.7, 12.7, 14.3, 15.5, 16.0, 17.2, 19.5, 20.6, 22.9; such as
   9.0, 10.3, 10.7, 12.7, 14.3, 15.5, 16.0, 17.2, 19.5, 20.6, 21.7, 22.3, 22.7, 22.9, 24.4.

In a further aspect the present invention provides crystalline 14-*O*-{[(1*R*,2*R*,4*R*)-4-amino-2-hydroxy-cyclohexylsulfanyl]-acetyl}-mutilin in the form of an L-lactate, Form 1, e.g. which is characterized by an X-ray powder diffraction pattern with peaks 2-theta at (degrees, ± 0.2, *inter alia*):
7.0, 11.6, 12.0, 12.5, 13.4, 13.6, 13.9, 15.3, 16.8, 18.8, 19.5, 19.8, 20.9, 23.3, 23.9, 24.2; such as
   7.0, 7.6, 11.6, 12.0, 12.5, 13.4, 13.6, 13.9, 15.3, 15.5, 16.8, 17.2, 18.8, 19.5, 19.8, 20.0, 20.9, 22.0, 22.4, 22.7, 23.3, 23.9, 24.2; 25.3, 28.9, 29.4, 30.8

In a further aspect the present invention provieds crystalline 14-*O*-{[(1*R*,2*R*,4*R*)-4-amino-2-hydroxy-cyclohexylsulfanyl]-acetyl}-mutilin in the form of a maleate, Form 1, e.g. which is characterized by an X-ray powder diffraction pattern with peaks 2-theta at (degrees, ± 0.2, *inter alia*):
7.0, 11.3, 11.7, 12.5, 13.5, 13.8, 15.3, 16.7, 18.3, 19.4, 19.7, 21.1, 22.2, 23.8, 23.9; such as 7.0, 11.3, 11.7, 12.5, 13.3, 13.5, 13.8, 14.1, 15.3, 16.7, 17.2, 18.0, 18.3, 19.4, 19.7,20.4, 21.1, 21.9, 22.2, 22.8, 23.8, 23.9, 24.9, 27.1, 27.8, 28.7, 29.3, 30.6, 30.8,

A compound of formula II in the form of a single stereoisomer may be prepared as appropriate, e.g. according or analogously to a method as conventional. Preferably a compound of formula II in the form of a single stereoisomer is prepared by coupling an amino-hydroxy-mercapto-cyclohexane, wherein the amino group is protected with an amino protecting group in the form of a single stereoisomer with an activated 14-O-acetyl-mutilin.

In another aspect the present invention provides a process for the production of a compound of formula II in the form of a single stereoisomer, comprising coupling a compound of formula 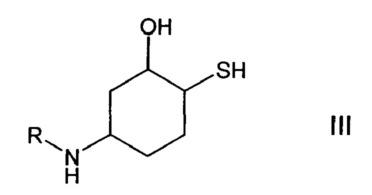 wherein R is as defined above, in the form of a single stereoisomer, e.g. including a compound of formula 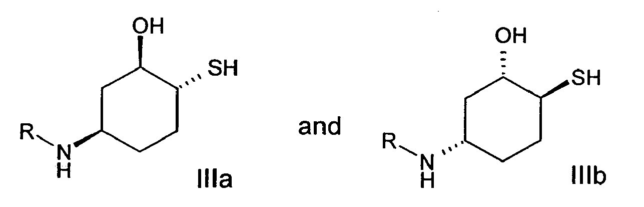 wherein R is as defined above, with an activated 14-O-AKT-acetyl-mutilin, wherein AKT is an activating group e.g mesyl, besyl or tosyl, preferably a tosyl group,
e.g. with an 14-O-tosyl-acetyl-mutilin of formula 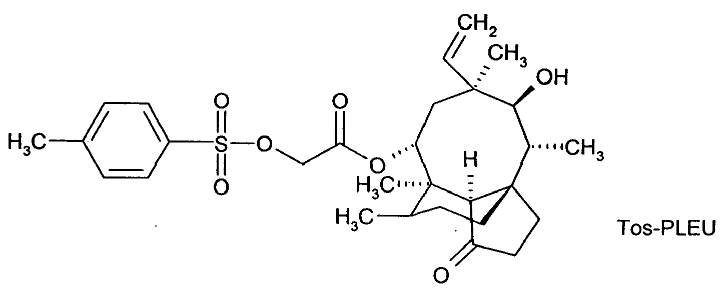 and isolating a compound of formula II obtained from the reaction mixture.

Compounds of formula III, including compounds of formula IIIa and IIIb, wherein R is as defined above are new and also form part of the present invention.

In another aspect the present invention provides a compound of formula III, e.g. in a single stereoisomeric form, such as a compound of formula IIIa or IIIb.

In a compound of formula IIIa the carbon atoms of the cyclohexyl ring to which the hydroxy group, the amine group and the thio group are attached are all in the R configuration and thus a compound of formula IIIa represents an optionally amino protected (1*R*,2*R*,4*R*)-4-amino-2-hydroxy-1-mercapto-cyclohexane; and in a compound of formula IIIb, respectively, the carbon atoms of the cyclohexyl ring to which the hydroxy group, the amine group and the thio group are attached are all in the S configuration and thus a compound of formula IIIb represents an optionally amino protected (1*S*,2*S*,4*S*)-4-amino-2-hydroxy-1-mercapto-cyclohexane.

The coupling reaction of a compound of formula III, wherein R is as defined above, in the form of a single stereoisomer with an activated 14-O-AKT-acetyl-mutilin, wherein AKT is as defined above, to obtain a compound of formula II, wherein R is as defined above in the form of a single stereoisomer may be performed as appropriate, e.g. according or analogously to a method as conventional, such as under standard conditions known for those reactions; e.g. in the presence of a base, e.g. a strong inorganic base, like a hydroxide such as NaOH, e.g. in a two phase system, and, if the reaction is performed in a two phase system preferably in the presence of a catalyst, such as a phase transfer catalyst, e. g. benzyl-tributylammonium chloride.

It has been found that during the coupling reaction the stereochemistry of the carbon atoms of the cyclohexyl moiety to which the thio, hydroxy and amino group are attached is retained and remains the same as in a compound of formula III used as a starting material.

Preferably the coupling reaction is performed in organic solvent, e.g. an apolar solvent, such as tert-butyl methyl ether (MTBE); preferably in an aqueous base solution, such as aqueous NaOH, preferably in the presence of a phase transfer catalyst, such as benzyl-tributylammonium chloride.

A compound of formula III in the form of a single stereoisomer may be prepared as appropriate, e.g. according, e.g. analogously to a method as conventional. Preferably a compound of formula III in the form of a single stereoisomer is prepared by deprotecting the thiol function in an amino-hydroxy-mercapto cyclohexane in the form of a single stereoisomer, wherein the amino group and the thiol group both are protected.

In another aspect the present invention provides a process for the preparation of a compound of formula III, comprising deprotecting the thiol function under reducing conditions e.g. by use of DIBAL, or under acidic conditions, or under basic conditions, e.g. by use of hydrazine hydrate, preferably by use of hydrazine hydrate in a compound of formula 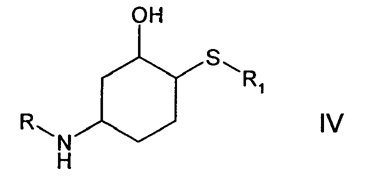 e.g. including a compound of formula 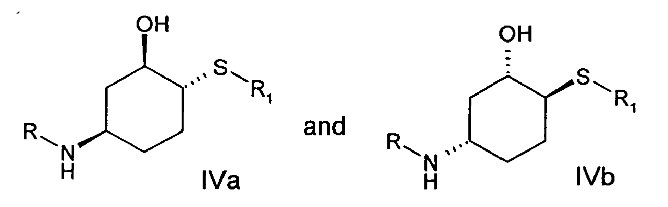 wherein R is as defined above and R₁ is a thiol protecting group in the form of a single stereoisomer,
and isolating a compound of formula III obtained from the reaction mixture.

A thiol protecting group, e.g. in the meaning of R₁ in a compound according to the present invention, includes e.g.
- (C₁₋₆)alkyl, wherein alkyl optionally is further substituted, e.g. further substiuted by (C₆₋₁₂)aryl such as phenyl, such as a trityl;
- -(C₁₋₆)alkylcarbonyl, e.g. acetyl,
- (C₆₋₁₂)arylcarbonyl, such as a benzoyl,
preferably -C(=O)-(C₆₋₁₂)aryl; more preferably benzoyl.

In a compound of formula IVa the carbon atoms of the cyclohexyl ring to which the hydroxy group, the amine group and the thio group are attached are all in the R configuration and thus a compound of formula IVa represents an amine and thio protected (1*R*,2*R*,4*R*)-4-amino-2-hydroxy-1-mercapto-cyclohexane; and in a compound of formula IVb the carbon atoms of the cyclohexyl ring to which the hydroxy group, the amine group and the thio group are attached are all in the S configuration and thus a compound of formula IVb represents an amino and thio protected (1*S*,2*S*,4*S*)-4-amino-2-hydroxy-1-mercapto-cyclohexane.

It has been found that during deprotection reaction of S-protected compounds of the formula IV the stereochemistry of carbon atoms of the cyclohexyl ring where a thio, hydroxy and amino group are attached in a compound of formula III obtained is retained and remains the same as in the compound of formula IV used as a starting material.

Deprotection of the thiol group is carried out as appropriate, e.g. by use of a cleaving agent, e.g. hydrazine hydrate. In order to minimize disulfide formation, deprotection is optionally performed in the presence of a reducing agent, e.g. dithiothreitol (DTT).

A compound of formula IV, e.g. including a compound of formula IVa and IVb, may be obtained as appropriate, e.g. analogously to a method as conventional or as described herein, e.g. in the example part.

In another aspect the present invention provides
- a process for the production of a compound of formula I in the form of a single stereoisomer, comprising using a compound of formula II in the form of a single stereoisomer, and/or comprising using a compound of formula III in the form of a single stereoisomer, and/or using a compound of formula IV in the form of a single stereoisomer as an intermediate, and
- a compound of formula II in the form of a single stereoisomer, and/or a compound of formula III in the form of a single stereoisomer, and/or a compound of formula IV in the form of a single stereoisomer for use as an intermediate in a process for the production of a compound of formula I in the form of a single stereoisomer.

In a further aspect the present invention provides a process for the production of a compound of formula I in the form of a single stereoisomer, comprising coupling a compound of formula III, wherein R is as defined above, in the form of a single stereoisomer with an activated 14-O-AKT-acetyl-mutilin, wherein AKT is an activating group, such as a mesyl, besyl or tosyl group, preferably a tosyl group e.g. a compound of formula Tos-PLEU, to obtain a compound of formula II, wherein R is as defined above,
optionally isolating a compound of formula II, wherein R is as defined above, in the form of a single stereoisomer obtained, from the reaction mixture,
deprotecting the amine function in a compound of formula II, wherein R is as defined above, in the form of a single stereoisomer obtained, and
isolating a compound of formula I, optionally in the form of a salt; from the reaction mixture, and, if desired,
converting a compound of formula I in the form of a single stereoisomer obtained in free form into a compound of formula I in salt form in the form of a single stereoisomer, or vice versa;
wherein optionally a compound of formula III, wherein R is as defined above in the form of a single stereoisomer is obtained by deprotection of the thiol function in a compound of formula IV, wherein R and R₁ are as defined above in the form of a single stereoisomer.

As an amine protecting group in a compound of formula II, III or IV conventional amino protecting groups may be used, preferably a tert-butoxycarbonyl group. If appropriate the amine protecting group R in a compound of formula II, III or IV may be changed, in a way that the carbon atom of the cyclohexyl ring to which the amine group is attached does not change its configuration, i.e. stereochemistry, e.g. via deprotection of the amine group, followed by protection with a different amine protecting group.

The process of the present invention enables the isolation of compounds of formula I in the form of single stereoisomers. The process controls stereochemistry and yields products as single stereoisomers whereby no sophisticated methods like chromatography e.g. normal phase or chiral phase e.g. chiral HPLC are necessary to separate the mixtures of diastereomers and regioisomers, e.g. as described in WO 2008/113089 which is very important for an industrially applicable process.

The present invention further relates to a novel crystalline forms of 14-*O*-{[(1*R*,2*R*,4*R*)-4-amino-2-hydroxy-cyclohexylsulfanyl]-acetyl}-mutilin in the form of an acetate, L-lactate or maleate. 14-*O*-{[(1*R*,2*R*,4*R*)-4-Amino-2-hydroxy-cyclohexylsulfanyl]-acetyl}-mutilin is converted to the crystalline salt forms using a crystallization process in organic medium. The process for the production of the novel crystalline salts may be enhanced and accelerated by the use of seed crystals. It has been found that the crystalline salts retain the stereochemistry of a compound of formula I in free form in the form of a single stereoisomer.

"In the form of a single stereoisomer" as used herein designates a form wherein the compound shows a diastereomeric or enantiomeric excess of ≥ 90% of the indicated stereochemistry.

The crystalline salts provided by the present invention are of desired and consistent chemical and optical purity have better stability compared to the amorphous lyophilized forms being advantageous in the storage and preparation of pharmaceutical compositions. No crystalline salt form of a compound of formula I, e.g. Ia has been described before , e.g. in WO 2008/113089.

Particularly preferred compounds of the present invention include the compounds of Examples 1 to 11, e.g. a compound selected from the group consisting of
*tert*-Butyl [(1*S*,3*S*,4*S*)-3-hydroxy-4-mercapto-cyclohexyl]-carbamate,
*tert*-Butyl [(1*R*,3*R*,4*R*)-3-hydroxy-4-mercapto-cyclohexyl]-carbamate, 14-*O*-{[(1*S*,2*S*,4*S*)-4-*tert*-Butoxycarbonylamino-2-hydroxy-cyclohexyl-sulfanyl]-acetyl}-mutilin,
14-*O*-{[(1*R*,2*R*,4*R*)-4-*tert*-Butoxycarbonylamino-2-hydroxy-cyclohexyl-sulfanyl]-acetyl}-mutilin,
14-*O*-{[(1*S*,2*S*,4*S*)-4-Amino-2-hydroxy-cyclohexylsulfanyl]-acetyl}-mutilin, 14-*O*-{[(1*R*,2*R*,4*R*)-4-Amino-2-hydroxy-cyclohexylsulfanyl]-acetyl}-mutilin, crystalline Form 1,
14-*O*-{[(1*R*,2*R*,4*R*)-4-Amino-2-hydroxy-cyclohexylsulfanyl]-acetyl}-mutilin, crystalline Form 2,
14-*O*-{[(1*R*,2*R*,4*R*)-4-Amino-2-hydroxy-cyclohexyl-sulfanyl]-acetyl}-mutilin acetate, crystalline Form A,
14-*O*-{[(1*R*,2*R*,4*R*)-4-Amino-2-hydroxy-cyclohexyl-sulfanyl]-acetyl}-mutilin acetate, crystalline Form B,
14-*O*-{[(1*R*,2*R*,4*R*)-4-Amino-2-hydroxy-cyclohexylsulfanyl] -acetyl} -mutilin L-lactate, crystalline Form 1, and
14-*O*-{[(1*R*,2*R*,4*R*)-4-Amino-2-hydroxy-cyclohexylsulfanyl]-acetyl}-mutilin maleate, in crystalline Form 1, and
14-*O*-{[(1*S*,2*S*,4*S*)-4-Amino-2-hydroxy-cyclohexyl-sulfanyl]-acetyl}-mutilin acetate.

### Description of the Figures

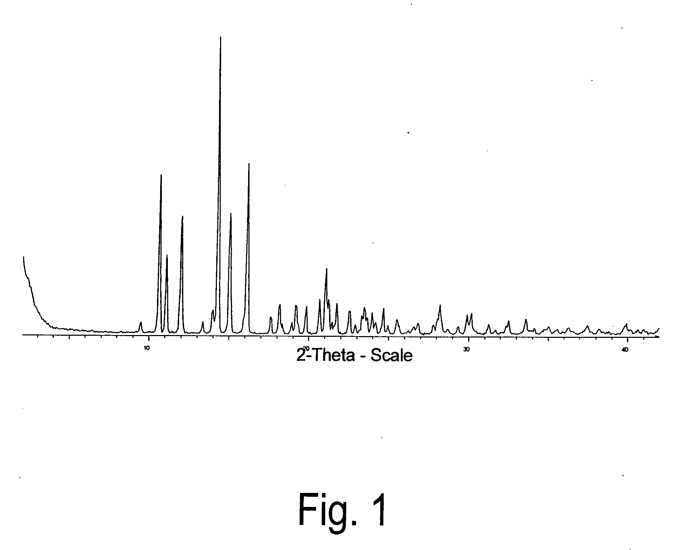

In Fig. 1 the Powder Diffractogram of 14-*O*-{[(1*R*,2*R*,4*R*)-4-amino-2-hydroxy-cyclohexylsulfanyl]-acetyl}-mutilin, Form 1 is indicated.

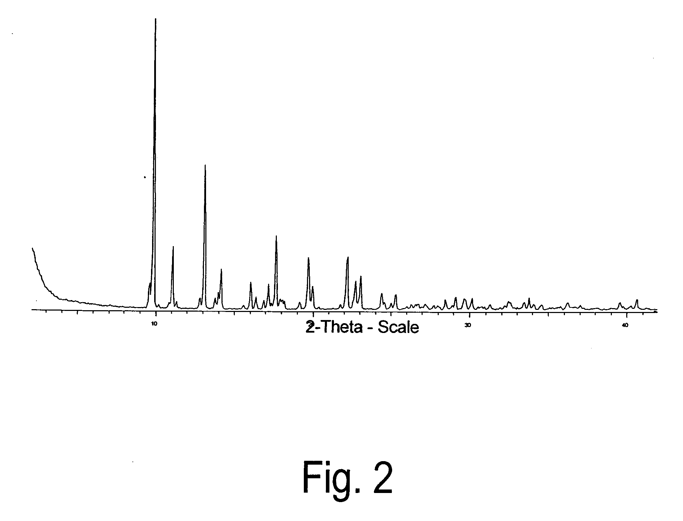

Fig. 2 shows the Powder diffractogram of 14-*O*-{[(1*R*,2*R*,4*R*)-4-amino-2-hydroxy-cyclohexylsulfanyl]-acetyl}-mutilin, Form 2.

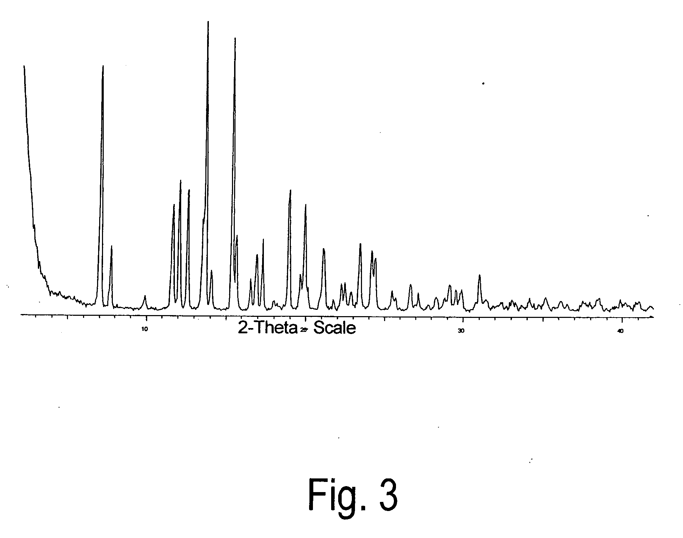

Fig. 3 shows the Powder diffractogram of 14-*O*-{[(1*R*,2*R*,4*R*)-4-amino-2-hydroxy-cyclohexylsulfanyl]-acetyl}-mutilin in the form of an acetate, Form A.

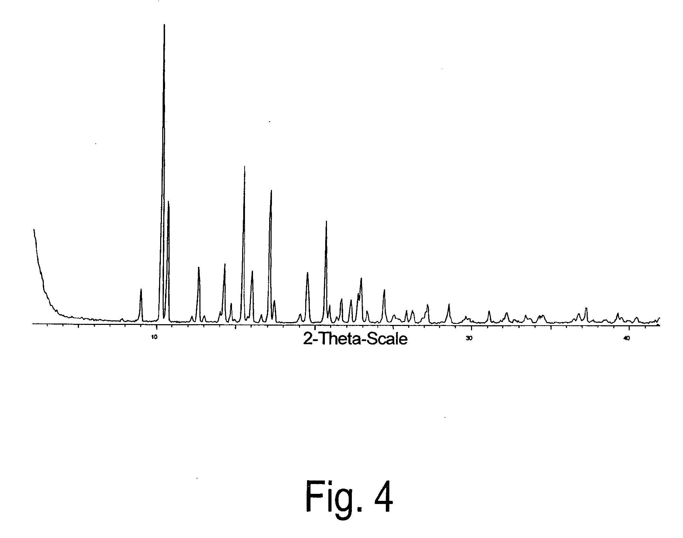

Fig. 4 shows the Powder diffractogram of 14-*O*-{[(1*R*,2*R*,4*R*)-4-amino-2-hydroxy-cyclohexylsulfanyl]-acetyl}-mutilin in the form of an acetate, Form B.

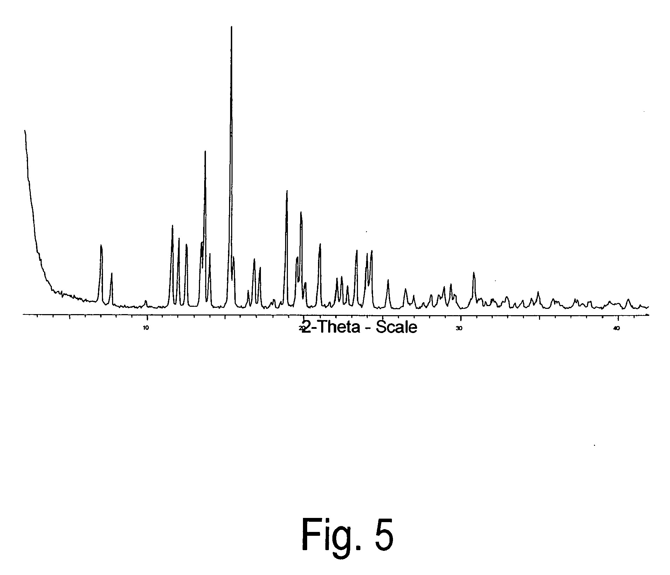

Fig. 5 shows the Powder diffractogram of 14-*O*-{[(1*R*,2*R*,4*R*)-4-amino-2-hydroxy-cyclohexylsulfanyl]-acetyl}-mutilin in the form of an L-lactate, Form 1.

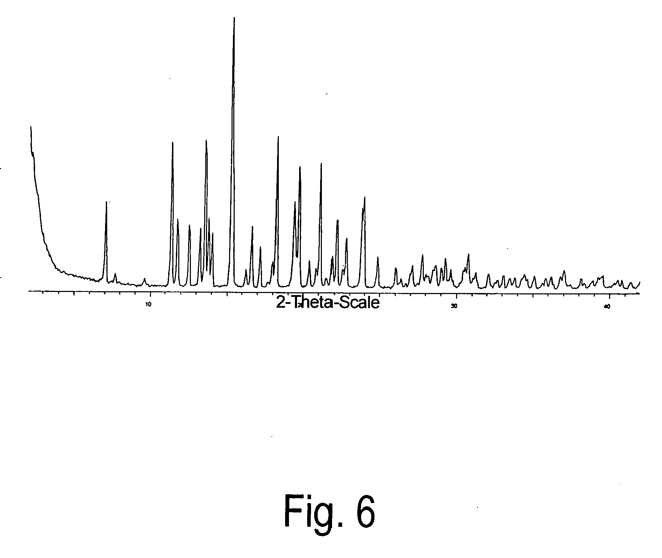

Fig. 6 shows the Powder diffractogram of 14-*O*-{[(1*R*,2*R*,4*R*)-4-amino-2-hydroxy-cyclohexylsulfanyl]-acetyl}-mutilin in the form of a maleate, Form 1.

The process for the synthesis of a compound of formula I, e.g. including compounds of formula Ia and Ib, is summarized in REACTION SCHEME 1:

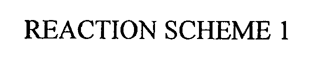

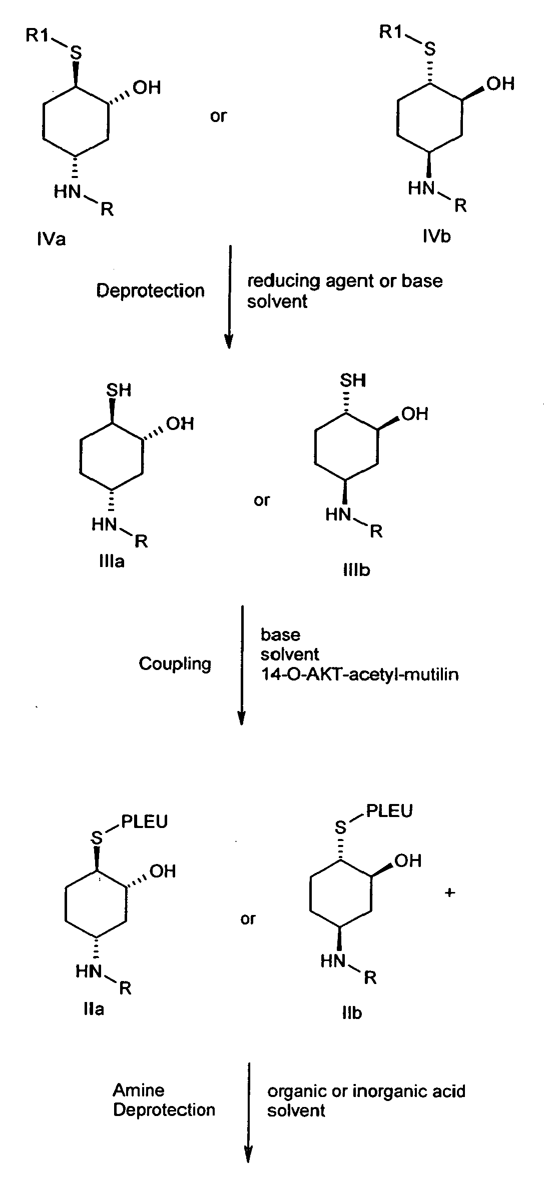

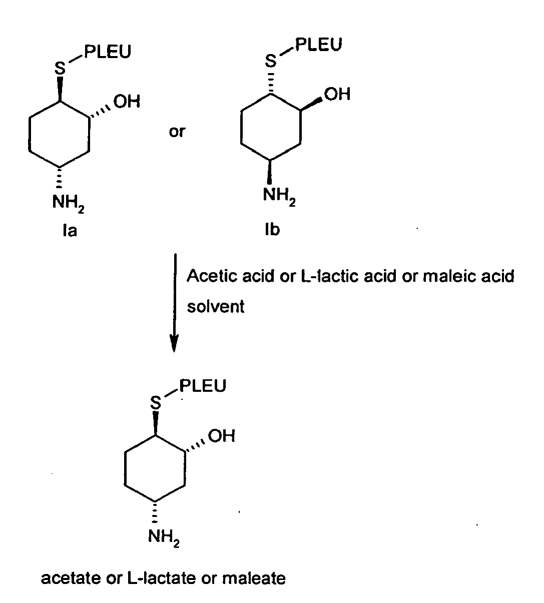

The synthesis of the starting materials of formula IV, e.g. of formula IVa or IVb, is outlined in Scheme 2 followed by the description of preparation.

Herein, including the examples and including SCHEME 1 and SCHEME 2 the following abbreviations are used:
- °C: degrees Celsius
- ¹H NMR: proton nuclear magnetic resonance spectroscopy
- ¹³C NMR: carbon nuclear magnetic resonance spectroscopy
- [α]_{D}: specific optical rotation angle at 589nm
- BOC: *tert*-butoxycarbonyl
- DCM: CH₂Cl₂
- DIBAL: diisobutylaluminium hydride
- DIPE: diisopropylether
- DMF: N,N-dimethylformamide
- DMSO: dimethylsulfoxide
- DMTF: dimethylthioformamide
- DPPA: diphenylphosphoryl azide
- DTT: 1,4-dithio-DL-threitol
- ESI+: electrospray ionization in positive mode
- EtOAc: ethyl acetate
- GF: glass fibre
- h: hours
- HPLC: High Performance Liquid Chromatography
- M: molarity
- mCPBA: metachloroperoxybenzoic acid
- MTBE: methyl tert-butyl ether
- min: minutes
- MS: mass spectrometry
- m/z: mass/charge ratio
- t-BuOH: tert-butylalcohol
- Bu₄NCl: tetrabutylammonium chloride
- PhCOSH: thiobenzoic acid
- rt: room temperature
- TLC: thin layer chromatography
- TEA, Et₃N: triethylamine
- TFA: trifluoroacetic acid
- THF: tetrahydrofuran
- Wt: weight
- XRPD: powder X-ray diffraction

Tos-PLEU.is a compound of formula

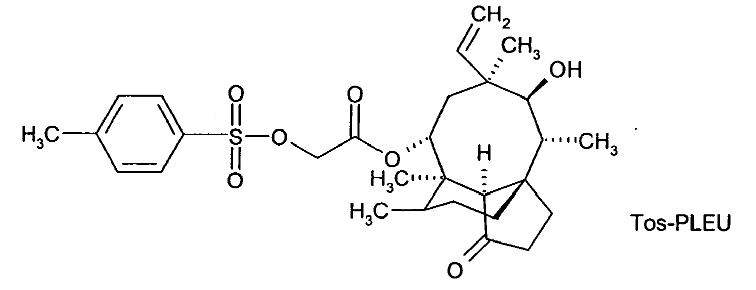

PLEU is a residue of formula

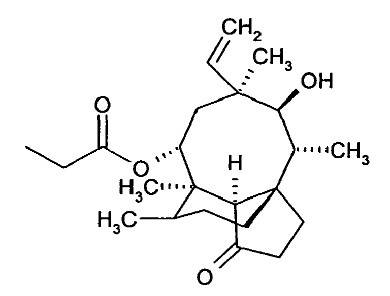

Any compound provided by the present invention is also designated herein as "a compound(s) of (according to) the present invention" and any process provided by the present invention is also designated herein as "a process(es) of (according to) the present invention".

Any compound provided according to the present invention may be obtained as appropriate, e.g. analogously to a method as conventional or as described herein.

In the following examples all temperatures are in degrees Celsius (°C) and are uncorrected. The abbreviations used are indicated above (after reaction SCHEME 1).

### Example 1

### tert-Butyl [(1S,3S,4S)-3-hydroxy-4-mercapto-cyclohexyl]-carbamate

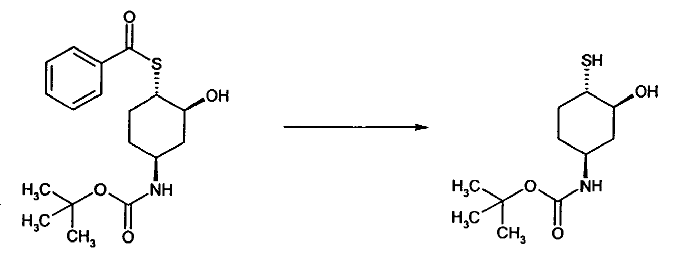

9.41 g of {(1*S*,2*S*,4*S*)-4-[(tert-Butoxycarbonyl)-amino]-2-hydroxy-cyclohexyl}-benzene-carbothioate and 94 mL of CH₂Cl₂ were charged to a 250 mL flask and stirred at 15-25°C. The mixture obtained was treated with argon for a period of 25 min (degassing). To the mixture obtained 0.94 g of 1,4-dithio-DL-threitol (10% wt) were added and 1.94 mL of hydrazine monohydrate dropwise. Upon completion of the reaction (HPLC control), 94 mL of 1M aqueous phosphoric acid solution were added to the mixture obtained over a period of 19 min and the mixture obtained was stirred for a further 10 min. Two phases formed were separated and the organic phase obtained was washed with 94 mL of 1M phosphoric acid followed by 94 mL of 1% aqueous NaCl solution. The mixture obtained was concentrated *in vacuo* at <40°C, to the concentration residue 94 mL of CH₂Cl₂ were added and the mixture again was concentrated. 6.64 g of *tert*-Butyl [(1*S*,3*S*,4*S*)-3-hydroxy-4-mercapto-cyclohexyl]-carbamate in the form of a white solid were obtained.
¹H NMR (200 MHz, DMSO-d₆, ppm) δ 6.79 (d, J=7.8Hz, 1H), 4.99 (d, J=5.8Hz, 1H), 3.34 - 3.26 (m, 1H), 3.12 - 3.04 (m, 1H), 2.37 (d, J=3.8Hz, 1H), 2.00 -1.89 (m, 1H), 1.87 - 1.82 (m, 1H), 1.73 - 7.67 (m, 1H), 1.47 - 1.04 (m, 12H)

### Example 2

### tert-Butyl [(1R,3R,4R)-3-hydroxy-4-mercapto-cyclohexyl]-carbamate

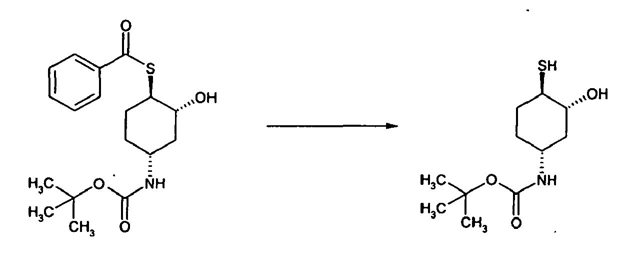

3940 g of {(1*R*,2*R*,4*R*)-4-[(*tert*-Butoxycarbonyl)-amino]-2-hydroxy-cyclohexyl}-benzene-carbothioate and 39.4 L of CH₂Cl₂ were charged to a vessel and stirred at 15-25°C. The mixture obtained was treated with argon for a period of 20 min (degassing). 394 g of 1,4-dithio-DL-threitol (10% wt) were added to the mixture obtained and 839.2 g of hydrazine monohydrate dropwise. The mixture obtained was stirred at 18 to 22°C for 3h and the reaction was followed by ¹H NMR. Upon completion of the reaction, 39.4 L of 10% aqueous phosphoric acid solution were added and the mixture obtained was stirred for a further 30 min. Two phases formed were separated and the organic phase obtained was washed with 39.4 L 1% aqueous NaCl solution. The organic layer was concentrated *in vacuo* at <40°C, to the concentration residue 39.4 L of CH₂Cl₂ were added and the mixture again was concentrated. 2890 g of *tert*-Butyl [(1*R*,3*R*,4*R*)-3-hydroxy-4-mercapto-cyclohexyl]-carbamate in the form of a white solid were obtained.
¹H NMR (200 MHz, DMSO-d₆, ppm) δ 6.79 (d, J=7.8Hz, 1H), 4.99 (d, J=5.8Hz, 1H), 3.34 - 3.24 (m, 1H, H-1), 3.14 - 3.04 (m, 1H), 2.37 (d, J=3.8 Hz, 1H), 2.00 -1.89 (m, 1H), 1.87-1.82 (m, 1H), 1.73 - 7.67 (m, 1H), 1.47 - 1.04 (m, 12H)

### Example 3

### 14-O-{[(1S,2S,4S)-4-tert-Butoxycarbonylamino-2-hydroxy-cyclohexyl-sulfanyl]-acetyl}-mutilin

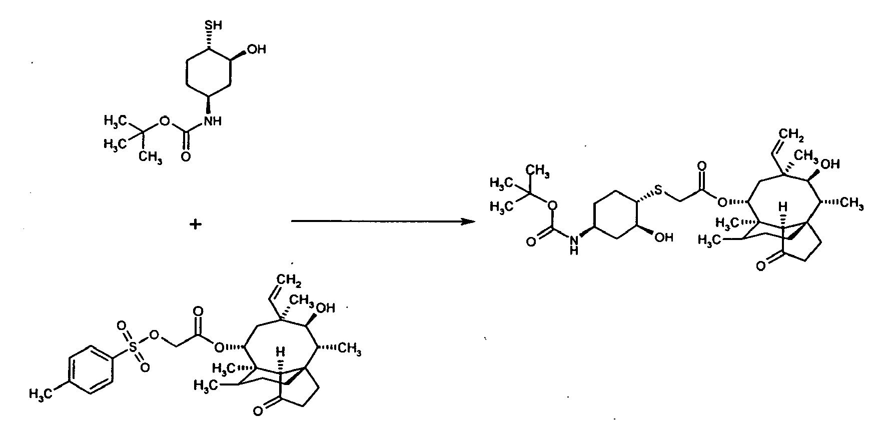

12.0 g of pleuromutilin tosylate (Tos-PLEU) and 120 mL of MTBE were charged into a 250 mL flask and the mixture obtained was treated with argon for 5 min at rt (degassing). To the mixture obtained 0.8 g of benzyl-tri-*n*-butylammonium chloride, 48 mL of 1M aqueous NaOH solution and 5.66 g of *tert*-Butyl [(1*S*,3*S*,4*S*)-3-hydroxy-4-mercapto-cyclohexyl]-carbamate were added with stirring. The mixture obtained was stirred at 20 to 25°C. Upon completion of the reaction (HPLC control) two layers formed were separated and the lower aqueous layer was removed. The organic phase obtained was washed with 1M aqueous NaOH solution, 1M phosphoric acid, 10% aqueous NaHCO₃ solution and water. The organic liquors obtained were dried and concentrated *in vacuo* at 35°C. 14.44 g of 14-*O-*{[(1*S*,2*S*,4*S*)-4-*tert*-Butoxycarbonylamino-2-hydroxy-cyclohexyl-sulfanyl]-acetyl}-mutilin in the form of a white foam were obtained.
¹H NMR (200 MHz, DMSO-d₆, ppm, *inter alia*) δ 6.78 (d, J=7.8Hz, 1H), 6.22 - 6.08 (m,1H), 5.55 (d, J=7.8Hz, 1H), 5.12 - 5.02 (m, 2H), 4.95 (d, J=5Hz, 1H), 4.52 (d, J=6Hz, 1H), 3.36 (AB, J=15Hz, 2H), 2.41 (s, broad, 1H), 1.36 (s, 12H), 1.06 (s, 3H), 0.81 (d, J=7Hz, 3H), 0.62 (d, J=6.6Hz, 3H)
MS (ESI+, g/mol): m/z 646 (MK⁺)

### Example 4

### 14-O-{[(1R,2R,4R)-4-tert-Butoxycarbonylamino-2-hydroxy-cyclohexyl-sulfanyl]-acetyl}-mutilin

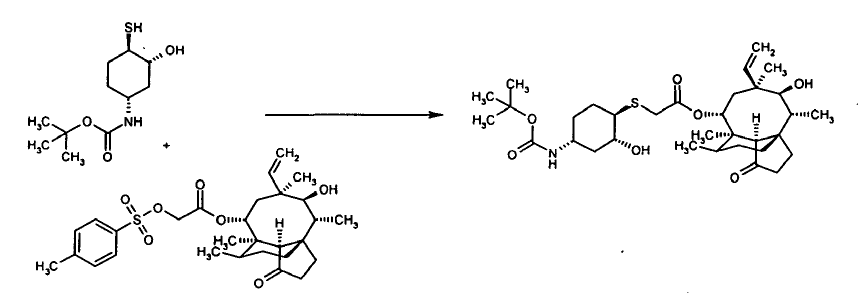

4750 g of pleuromutilin tosylate (Tos-PLEU) and 49.3 L of MTBE were charged into a vessel and the mixture obtained was treated with argon (degassing). To the mixture obtained 311.5 g of benzyl-tri-*n*-butylammonium chloride, 19.8 L of 1M aqueous NaOH solution and 2316 g of *tert*-Butyl [(1*R*,3*R*,4*R*)-3-hydroxy-4-mercapto-cyclohexyl]-carbamate were added with stirring. The mixture obtained was stirred at 17 to 23°C for 3 h. Upon completion of the reaction (¹H NMR control) two layers formed were separated and the lower aqueous layer was removed. The organic phase obtained was washed with 1M aqueous NaOH solution, 1M phosphoric acid, 10% aqueous NaHCO₃ solution and water. The organic liquors obtained were concentrated and dried *in vacuo* at 35°C. 5090 g of 14-*O*-{[(1*R*,2*R*,4*R*)-4-*tert-*Butoxycarbonylamino-2-hydroxy-cyclohexyl-sulfanyl]-acetyl}-mutilin in the form of a white foam were obtained.
¹H-NMR (200 MHz, DMSO-d₆, ppm, *inter alia*) δ 6.78 (d, J=7.8Hz, 1H), 6.22 - 6.08 (m,1H), 5.55 (d, J=7.8Hz, 1H), 5.13 - 5.02 (m, 2H), 4.95 (d, J=5Hz, 1H), 4.52 (d, J=6Hz, 1H), 3.36 (AB, J=15Hz, 2H), 2.40 (s, broad, 1H), 2.15 - 2.0 (m, 3H), 1.9 - 1.8 (m, 1H), 1.35 (s, 9H), 0.81 (d, J=7Hz, 3H), 0.62 (d, J=6.6Hz, 3H)
MS (ESI+, g/mol): m/z 653 (M+2Na⁺)

### Example 5

### 14-O-{[(1S,2S,4S)-4-Amino-2-hydroxy-cyclohexylsulfanyl]-acetyl}-mutilin

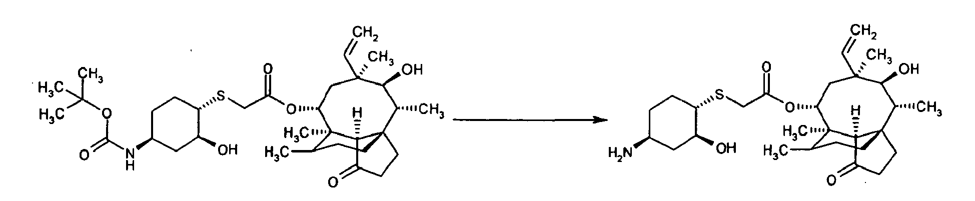

14.44 g of 14-*O*-{[(1*S*,2*S*,4*S*)-4-*tert*-Butoxycarbonylamino-2-hydroxy-cyclohexyl-sulfanyl]-acetyl}-mutilin and 29 mL of isopropanol were charged into a 100 mL flask and stirred. To the mixture obtained, 14 mL of 85% phosphoric acid were added and the reaction temperature was raised to ca. 35°C. The mixture obtained was heated to 50°C, stirred overnight and analysed for rection completion by HPLC. Upon completion of the reaction the mixture was cooled to rt, transferred to a 250 mL flask and 144 mL of CH₂Cl₂ were added. The mixture obtained was cooled to 0 to 5°C and 144 mL of 30% aqueous Na₂CO₃ solution were added slowly. The mixture obtained was warmed to rt, two phases obtained were separated, the aqueous phase was extracted with CH₂Cl₂ and the combined organic phases were washed with water. The mixture obtained was concentrated to approximately 5 volumes, 72 mL of CH₂Cl₂ were added and the mixture obtained again was concentrated to approximately 5 volumes and that procedure was repeated once more. To the concentration residue obtained 144 mL of di-*iso*propyl ether were added dropwise over a period of 1 h and the mixture obtained was concentrated. 11.19 g of 14-*O*-{[(1*S*,2*S*,4*S*)-4-Amino-2-hydroxy-cyclohexylsulfanyl]-acetyl}-mutilin in the form of a solid were obtained.
The crude material obtained was subjected to purification by column chromatography over silica gel (eluent: EtOH-EtOAc 5:1 (+1% aq.NH₄OH solution (25%)) to yield 8.71 g of pure 14-*O*-{[(1*S*,2*S*,4*S*)-4-Amino-2-hydroxy-cyclohexylsulfanyl]-acetyl}-mutilin in the form of a solid.
¹H NMR (200 MHz, DMSO-d₆, ppm, *inter alia*) δ 6.22 - 6.08 (m, 1H), 5.55 (d, J=8Hz, 1H), 5.21 - 5.01 (m, 2H), 4.05 (s, very broad, 4H), 3.38 (AB, J=15Hz, 2H), 2.8 - 2.6 (m, 1H), 2.41 (s, broad, 1H), 1.36 (s, 3H), 1.05 (s, 3H), 0.82 (d, J=7Hz, 3H), 0.63 (d, J=8Hz, 3H)
MS (ESI+, g/mol): m/z 508 (MH⁺)

### Example 6

### 14-O-{[(1R,2R,4R)-4-Amino-2-hydroxy-cyclohexylsulfanyl]-acetyl}-mutilin, crystalline Form 2

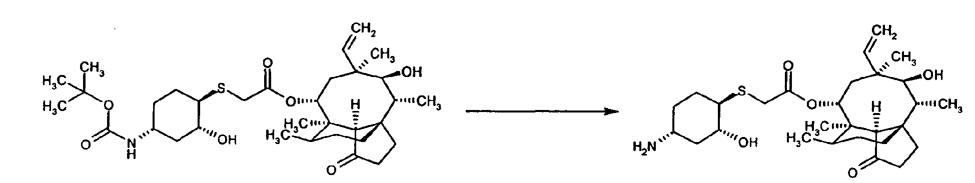

5060 g of 14-*O*-{[(1*R*,2*R*,4*R*)-4-*tert*-Butoxycarbonylamino-2-hydroxy-cyclohexyl-sulfanyl]-acetyl}-mutilin and 10.12 L of isopropanol were charged into a vessel and stirred at 20 to 25°C. 5.06 L of 85% phosphoric acid were added and the reaction temperature was raised to ca. 35°C. The mixture obtained was heated to 47 to approx. 50°C for 16 h and analysed for reaction completion by HPLC. Upon completion of the reaction the mixture was cooled to 20 to 25°C 50.5 L of CH₂Cl₂ were added. The mixture obtained was cooled to 0 to 5°C and 50.6 L of 30% aqueous K₂CO₃ solution were added over 1 h at <25°C. The mixture obtained was warmed to rt, two phases obtained were separated, the aqueous phase was extracted with CH₂Cl₂ and the combined organic phases were washed with water. The mixture obtained was concentrated to approximately 5 volumes, 25.3 L of CH₂Cl₂ were added and the mixture obtained again was concentrated to approximately 5 volumes and that procedure was repeated once more but the mixture was concentrated to ca. 2 volumes. The concentration residue obtained was cooled to 18 to 22°C and 50.6 L of di-*iso*propyl ether were added dropwise over a period of 1 h. The slurry obtained was stirred at 15 to 25°C for a minimum of 2 h, filtered and the solid obtained was washed with 10.1 L of di-*iso*propyl ether and was dried. Crude 14-*O*-{[(1*R*,2*R*,4*R*)-4-Amino-2-hydroxy-cyclohexylsulfanyl]-acetyl}-mutilin was obtained.

For further purification crude 14-*O*-{[(1*R*,2*R*,4*R*)-4-Amino-2-hydroxy-cyclohexylsulfanyl]-acetyl}-mutilin and 5 volumes of n-butanol were heated to 88 to 92°C until complete dissolution. The mixture obtained was allowed to cool to 40 to 45°C over at least 2 h and further stirred at this temperature for 2 h. The mixture was filtered and the precipitate obtained was washed with *n*-butanol followed by *tert*-butyl methyl ether. That purification procedure was repeated and the resultant product was dried *in vacuo* at <40°C. 3260 g of crystalline 14-*O*-{[(1*R*,2*R*,4*R*)-4-amino-2-hydroxy-cyclohexylsulfanyl]-acetyl}-mutilin, Form 2, were obtained in the form of a white solid.
¹H NMR (400 MHz, CDCl₃, ppm, *inter alia*) δ 6.51 - 6.44 (m, 1H), 5.78 (d, J=8Hz, 1H), 5.38 - 5.20 (m, 2H), 3.48 - 3.40 (m, 1H), 3.36 (d, J=7Hz, 1H), 3.25 (AB, J=15Hz, 2H), 2.92 - 2.82 (m, 1H), 2.6 - 2.5 (m, 1H), 1.45 (s, 3H), 1.20 (s, 3H), 0.88 (d, J=7Hz, 3 H), 0.73 (d, J=8Hz, 3H)
MS (ESI+, g/mol): m/z 508 (MH⁺)

### Example 7

### 14-O-{[(1R,2R,4R)-4-Amino-2-hydroxy-cyclohexylsulfanyl]-acetyl}-mutilin, crystalline Form 1

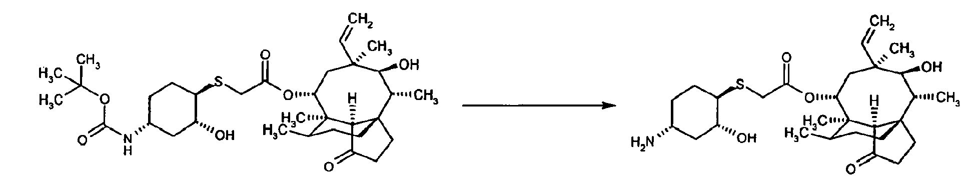

To a solution of 900 g of 14-*O*-{[(1*R*,2*R*,4*R*)-4-*tert*-Butoxycarbonylamino-2-hydroxycyclohexyl-sulfanyl]-acetyl}-mutilin in 9 L dichloromethane at 15 to 25°C was charged 1.8 L trifluoroacetic acid at 15 to 25°C and the resulting solution was stirred for 2 hours. Following reaction completion the reaction mixture was concentrated under vacuum and the residue azeo-dried with a total of 9 L dichloromethane. The concentrate obtained was dissolved in 4.5 L of dichloromethane, the solution cooled to 0 to 5°C and the pH adjusted to pH 11 with aqueous 3.6 L potassium carbonate (2.5M). The biphasic mixture obtained was warmed to 15 to 20°C and stirred for 5 to 10 minutes. The layers were separated, the aqueous phase extracted with 1.8 L dichloromethane, the organic phases combined, washed with 2.3 L water, dried over sodium sulphate, filtered, the collected solids washed with 0.9 L dichloromethane and the combined filtrates concentrated to dryness under vacuum at <40°C to yield crude 744 g 14-*O*-{[(1*R*,2*R*,4*R*)-4-Amino-2-hydroxy-cyclohexyl-sulfanyl]-acetyl}-mutilin.

For further purification the following procedure was applied:

To crude 744 g 14-*O*-{[(1*R*,2*R*,4*R*)-Amino-2-hydroxy-cyclohexyl-sulfanyl]-acetyl}-mutilin were added 2.23 L tetrahydrofuran and the resulting suspension was stirred at 15 to 25°C for 60 min. To the mixture obtained 7.44 L MTBE were added over 15 to 30 min, the suspension obtained was aged for 60 min and filtered under nitrogen. The collected solids were washed with a total of 3 L MTBE and pulled dry on the filter under nitrogen for 1.5 hours to give 626 g of 14-*O*-{[(1*R*,2*R*,4*R*)-4-Amino-2-hydroxy-cyclohexyl-sulfanyl]-acetyl}-mutilin. The isolated 14-*O*-{[(1*R*,2*R*,4*R*)-4-Amino-2-hydroxy-cyclohexyl-sulfanyl]-acetyl}-mutilin conforms to crystalline Form 1.

### Example 8

### 14-O-{[(1R,2R,4R)-4-Amino-2-hydroxy-cyclohexyl-sulfanyl]-acetyl}-mutilin acetate, crystalline Form A

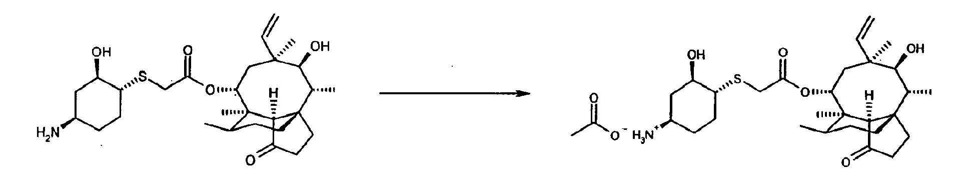

To a suspension of 615 g 14-*O*-{[(1*R*,2*R*,4*R*)-4-Amino-2-hydroxy-cyclohexyl-sulfanyl]-acetyl}-mutilin in 12.3L of methyl acetate at 50 to 55°C were added 62 ml of water and the resulting hazy solution was clarified through GF filter paper. The filtrates obtained were heated to 50 to 55°C (clear solution), 123 ml acetic acid were added, the resultant mixture was stirred at 50 to 55°C for 25 minutes, cooled to 15 to 25°C over 80 minutes and further cooled to 0 to 5°C over 60 min. The resulting suspension was aged at 0 to 5°C for 80 minutes, filtered and the filter-cake was washed with 3.08 L of methyl acetate. The filter-cake was pulled dry on the filter under nitrogen for 2 hours to give 574.1 g 14-*O-*{[(1*R*,2*R*,4*R*)-4-Amino-2-hydroxy-cyclohexyl-sulfanyl]-acetyl}-mutilin in the form of an acetate in the form of a crystalline, fine white powder. The obtained polymorphic form corresponds to Form A.

The ¹H NMR pattern is identical with that of example 9.

### Example 9

### 14-O-{[(1R,2R,4R)-4-Amino-2-hydroxy-cyclohexyl-sulfanyl]-acetyl}-mutilin acetate, crystalline Form B

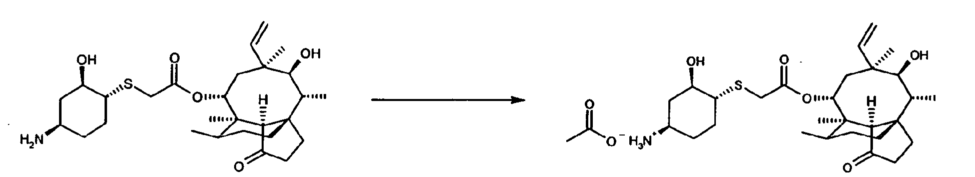

To a suspension of 3260 g 14-*O*-{[(1*R*,2*R*,4*R*)-4-Amino-2-hydroxy-cyclohexyl-sulfanyl]-acetyl}-mutilin in 56.8 L of *i*-PrOAc were added 10 g of seed crystals of 14-*O*-{[(1*R*,2*R*,4*R*)-4-amino-2-hydroxy-cyclohexyl-sulfanyl]-acetyl}-mutilin (Form B). The suspension was stirred at 20 to 25 °C for 10 min. To the mixture obtained were added 353 ml of acetic acid, and the mixture obtained was stirred at 20 to 25 °C for 1 h and tested for completion and polymorphic form by XRPD. The suspension obtained was stirred for a further 1 h at 20 to 25 °C, filtered, and the filter-cake was washed with 5.68 L of *i*-PrOAc. The solid obtained was dried under vacuum at 50°C for at least 12 h and sieved provide 3.15 kg of 14-*O-*{[(1*R*,2*R*,4*R*)-4-amino-2-hydroxy-cyclohexyl-sulfanyl]-acetyl}-mutilin acetate in the form of a white solid. The isolated 14-*O*-{[(1*R*,2*R*,4*R*)-4-Amino-2-hydroxy-cyclohexyl-sulfanyl]-acetyl}-mutilin acetate conforms to crystalline Form B.
¹H NMR (500 MHz, DMSO-d₆, ppm, *inter alia*) δ 6.16 - 6.10 (m, 1 H), 5.54 (d, J=8.3Hz, 1H), 5.09 - 5.02 (m, 2H), 3.42 (d, J=6Hz, 1H), 3.37 (AB, J=15Hz, 2H), 3.29 - 3.25 (m, 1H), 2.77 - 2.67 (m, 1H), 2.55 - 2.5 (m, 1H), 2.40 (s, broad, 1H), 2.23 - 2.12 (m, 1H), 2.12 - 2.03 (m, 3H), 2.03 - 1.95 (m, 1H), 1.94 - 1.85 (m, 1H), 1.77 (s, 3H), 1.77 - 1.71 (m, 1H), 1.7 - 1.57 (m, 2H), 1.52 - 1.43 (m, 1H), 1.43 -1.37 (m, 1H), 1.36 (s, 3H), 1.37 - 0.96 (m, 10H), 0.81 (d, J=7Hz, 3H), 0.62 (d, J=7Hz, 3H)

### Example 10

### 14-O-{[(1R,2R,4R)-4-Amino-2-hydroxy-cyclohexylsulfanyl]-acetyl}-mutilin L-lactate, crystalline Form 1

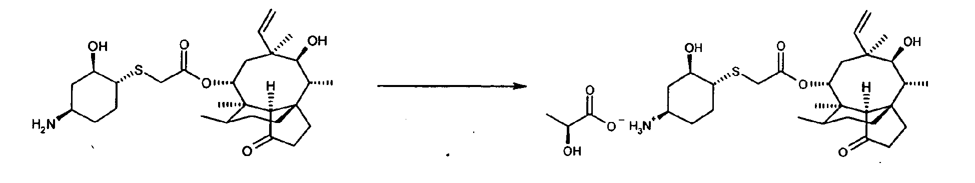

22 g 14-*O*-{[(1*R*,2*R*,4*R*)-4-Amino-2-hydroxy-cyclohexyl-sulfanyl]-acetyl}-mutilin and 62.5 L of ethyl acetate were charged to a vessel. The suspension obtained was heated to 50°C and held until dissolution. 1 eq of 98% L-lactic acid was charged to the mixture obtained, and the mixture obtained was gradually cooled to 25°C over 3h, during which 14-*O*-{[(1*R*,2*R*,4*R*)-4-Amino-2-hydroxy-cyclohexyl-sulfanyl]-acetyl}-mutilin L-lactate seed crystals were added. The resultant suspension was stirred at 20-25°C overnight and further cooled to 5°C for 1h. The 14-*O*-{[(1*R*,2*R*,4*R*)-4-Amino-2-hydroxy-cyclohexyl-sulfanyl]-acetyl}-mutilin in the form of an L-lactate salt was isolated by filtration and dried in vacuo at 40°C overnight giving 23.7 g of product as a white, crystalline solid. The isolated material corresponds to crystalline Form 1.
¹H-NMR (400 MHz, DMSO-d₆, ppm, *inter alia*) δ 6.13 (dd, J=11 and 18Hz, 1H), 5.54 (d, J=8Hz, 1H), 5.10 - 5.01 (m, 2H), 4.53 (d, broad, 1H), 3.60 (dd, J=7 and 14Hz, 1H), 3.40 (AB, J=15Hz, 2H), 2.93 (m, 1H), 2.55 - 2.48 (m, 1H), 2.39 (s, broad, 1H), 1.36 (s, 3H), 1.09 (d, J=7Hz, 3H), 1.04 (s, 3H), 0.81 (d, J=7Hz, 3H), 0.61 (d, J=7Hz, 3H).

### Example 11

### 14-O-{[(1R,2R,4R)-4-Amino-2-hydroxy-cyclohexylsulfanyl]-acetyl}-mutilin maleate, crystalline Form 1

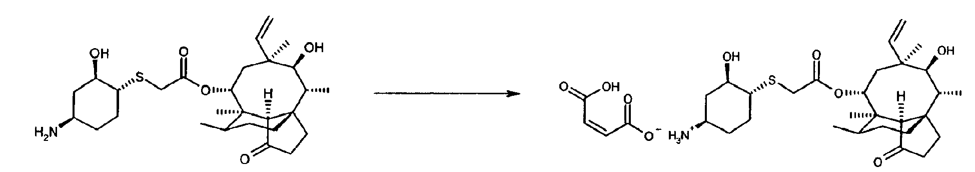

5.5 g 14-*O*-{[(1*R*,2*R*,4*R*)-4-Amino-2-hydroxy-cyclohexyl-sulfanyl]-acetyl}-mutilin and 110 ml of ethyl acetate were charged to a flask. The suspension obtained was heated to 80°C and held until dissolution. To the mixture obtained 10.8 ml of maleic acid were charged, and the mixture obtained was allowed to cool to rt overnight with stirring. 6.16 g of 14-*O-*{[(1*R*,2*R*,4*R*)-4-Amino-2-hydroxy-cyclohexyl-sulfanyl]-acetyl}-mutilin in the form of a crystalline maleate salt were isolated by filtration and dried for 6 h in vacuo. The isolated material corresponds to crystalline Form 1.
¹H NMR (400 MHz, DMSO-d-₆, ppm, *inter alia*) δ 6.13 (dd, J=11 and 18Hz, 1H), 6.00 (s, 2H), 5.54 (d, J=8Hz, 1H), 5.10 - 5.01 (m, 2H), 4.54 (d, J=6Hz, 1H), 3.40 (AB, J=15Hz, 2H), 3.05 (m, 1H), 2.56 - 2.49 (m, 1H), 2.40 (s, broad, 1H), 1.36 (s, 3H), 1.05 (s, 3H), 0.81 (d, J=7Hz, 3H), 0.61 (d, J=7Hz, 3H).

### Example 12

### 14-O-{[(1S,2S,4S)-4-Amino-2-hydroxy-cyclohexyl-sulfanyl]-acetyl}-mutilin acetate

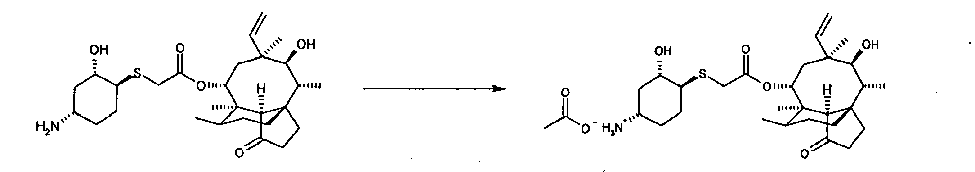

To a suspension of 7 g 14-*O*-{[(1*S*,2*S*,4*S*)-4-Amino-2-hydroxy-cyclohexyl-sulfanyl]-acetyl}-mutilin in 140 ml of *i*-PrOAc were added 0.87 ml of acetic acid. The suspension obtained was stirred for 2 h at 20 to 25 °C, filtered, and the filter-cake was washed with 14 ml of *i-*PrOAc. The solid obtained was dried under vacuum < 40°C. 7.46 g of 14-*O*-{[(1*S*,2*S*,4*S*)-4-amino-2-hydroxy-cyclohexyl-sulfanyl]-acetyl}-mutilin acetate were isolated in the form of a white solid.
¹H NMR (500 MHz, DMSO-d₆, ppm, *inter alia*) δ 6.22 - 6.07 (m, 1 H), 5.55 (d, J=8.0 Hz, 1H), 5.11 - 5.02 (m, 2H), 3.51 - 3.25 (m, 4H), 2.80 (m, 1H), 2.54 - 2.50 (m, 1H), 2.40 (s, broad, 1H), 2.27 - 2.03 (m, 4H), 1.97 - 0.95 (m, 23H), 0.82 (d, J=7.8 Hz, 3H), 0.63 (d, J=5.8 Hz, 3 H)

The process to the starting materials of formula IVa and IVb useful for the production of compounds of formula IIIa and IIIb is summarized in Reaction Scheme 2 below. 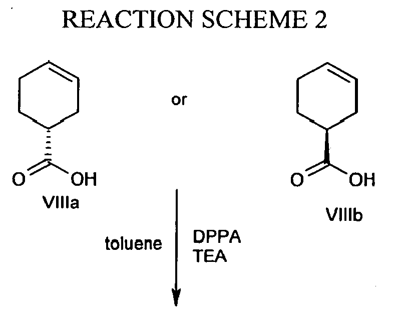 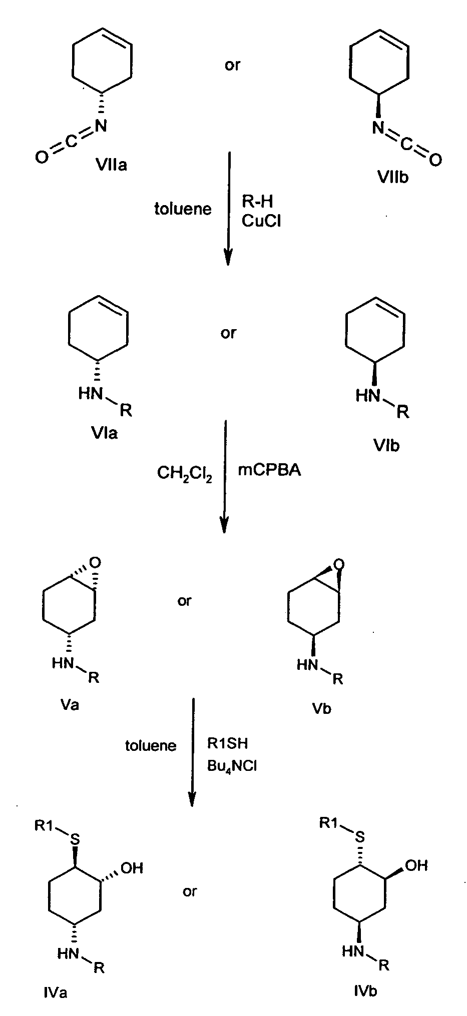 wherein R represents an amino protecting group and and R₁ represents a sulfur protecting group and are defined as above.

### Example 13

### tert-Butyl cyclohex-3-enyl-1(R)-carbamate

### A. Salt Formation of cyclohex-3-ene-1-carboxylic acid

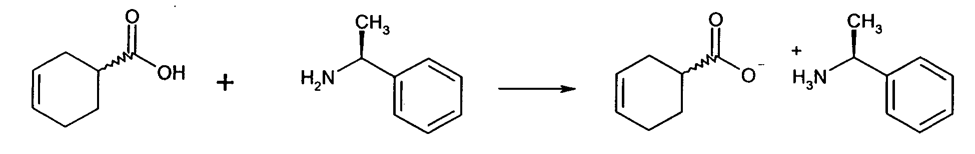

1000 g of racemic cyclohex-3-ene-1-carboxylic acid were charged to a flask and 5 volumes of acetone were added. The mixture obtained was stirred, heated to 55 to 60°C and stirred for 30 min. To the mixture obtained 960.5 g of (*S*)-(-)-methylbenzylamine in 2 volumes of acetone were added dropwise over approximately 25 min. A clear, orange solution was obtained and cooled slowly. Crystallisation started at 53°C (after 30 min). Full crystallisation occurred after ~1h at 49°C. The mixture obtained was cooled to rt over a further 3 h with an ice bath then stirred at rt for a further 1.5 h. The precipitate obtained was filtered off and washed with acetone. A methylbenzylamine salt of cyclohex-3-ene-1-carboxylic acid as set out in the reaction scheme above was obtained.
Yield (wet): 1966.9 g; optical rotation: ²⁰[α]_{D} = +8.05° (c=1, MeOH)

### B. Salt Resolution

1966.9 g (wet) of a salt as set out under step A and 3.8 volumes of acetone were charged to a 10 L vessel and heated to 55 to 60°C. When the product had dissolved, the reaction was stirred for a further 15 min and then slowly cooled to rt. Crystallisation started after 1 h 10 min (53°C). The mixture obtained was cooled to 20 to 25°C over 4.5 h and stirred at rt for a further 1.5 h. The precipitate obtained was filtered off and washed with acetone. A methylbenzylamine salt of cyclohex-3-ene-1-carboxylic acid wherein the R-isomer was enriched was obtained.
Yield (wet): 1143g; optical rotation: ²⁰[α]_{D} = +20.65° (c=1, MeOH)

Step B. was repeated until a required optical rotation (²⁰[α]_{D} > 40°) was achieved.

### C. Cyclohex-3-ene-1(R)-carboxylic acid

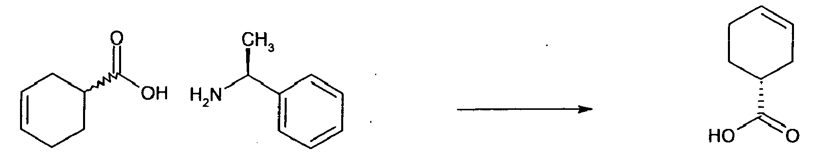

579.6 g of cyclohex-3-ene-1(*R*)-carboxylic acid (S)-(-)-methylbenzylamine salt and 5 volumes of MTBE were charged to a flask at 20-25°C and stirred. To the mixture obtained 10 volumes of 1M HCl were added, the mixture obtained was stirred for 5-10 min and two layers were formed. The layers obtained were separated and the aqueous layer was extracted with MTBE. The organic layers obtained were combined and washed with brine. The organic phase obtained was dried over Na₂SO₄, filtered, and the filter cake obtained was washed with MTBE. From the filtrate obtained solvent was removed in vacuo. cyclohex-3-ene-1(*R*)-carboxylic acid in the form of a clear oil was obtained.
Yield: 301.78 g
Optical rotation ²⁰[α]_{D} =+83.1° (c=1, CHCl₃)

Cyclohex-3-ene-1(*R*)-carboxylic acid may be obtained in analogy to the method disclosed in Schwartz, H. M.; et al. JACS 1978, 100, 5199-5203.

### D. Curtius Rearrangement

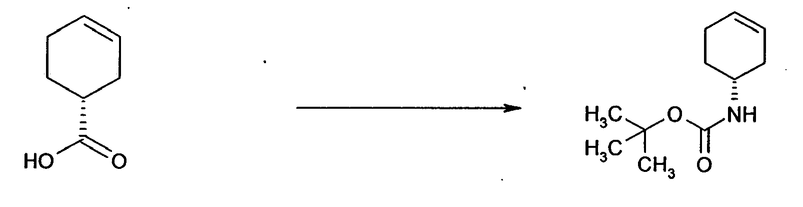

305 g of cyclohex-3-ene-1(*R*)-carboxylic acid and 10 volumes of toluene were charged to a flask at 20-25°C and stirred. To the mixture obtained 1.1 equivalents of NEt₃ were added dropwise over 15 min and the mixture obtained was stirred for a further 20 min. To the mixture obtained 1.0 equivalents of DPPA were added dropwise over approximately 20 minutes and the temperature raised to 95°C (exothermic reaction) with vigorous gas evolution. The mixture obtained was stirred for 15 min and heated to reflux. Progress of the reaction was followed by ¹H NMR measurements until completion. The mixture obtained was cooled to 80°C over 35 min and 5 equivalents of tert-butanol were added dropwise over 10 min, followed by 0.04 equivalents of CuCl. The mixture obtained was warmed to 100°C and stirred for a further 40 min. Progress of the reaction was followed by ¹H NMR measurements until completion. The mixture obtained was cooled and 5 volumes of aqueous, saturated NaHCO₃ solution were added over 10 min. The mixture obtained was stirred for 20 min and left overnight. The mixture obtained was filtered and the residual solid was washed with toluene. The organic layers were separated and the aqueous layer was washed with toluene. All organic layers obtained were combined, washed with H₂O and solvent was removed *in vacuo*. *tert*-Butyl cyclohex-3-enyl-1(*R*)-carbamate was obtained in the form of a light brown solid. Crude Yield: 479.7 g

The crude *tert*-Butyl cyclohex-3-enyl-1(*R*)-carbamate obtained was subjected to chromatography. For 160 g of crude product the column was packed with 1.5 Kg silica gel, using 2.5 L of cyclohexane, and topped with sand. The crude product was loaded in 0.8 L of 5% EtOAc/cyclohexane. The column was flashed with the following gradient system, a discrete fraction being collected each time:
2% EtOAc/cyclohexane (9 x 0.8L fractions)
5% EtOAc/ cyclohexane (7 x 0.8L fractions)
10% EtOAc/ cyclohexane (4 x 0.8L fractions)
Overall yield after chromatography: 81.3% of theory
¹H NMR (CDCl₃, 500 MHz, ppm): δ 5.64-5.67 (m, 1H), 5.56-5-60 (m, 1H), 4.54 (s, broad, 1H), 3.77 (s, broad, 1H), 2.32-2.34 (m, 1H), 2.07-2.17 (m, 2H), 1.81-1.87 (m, 2H), 1.48-1.56 (m, 1H), 1.44 (s, 9H)
¹³C NMR (CDCl₃, 500 MHz, ppm):): δ 155.3, 126.9, 124.5, 79.1, 45.7, 32.1, 28.4, 23.6

### Example 14

### tert-Butyl cyclohex-3-enyl-1(S)-carbamate

### A. Salt Formation

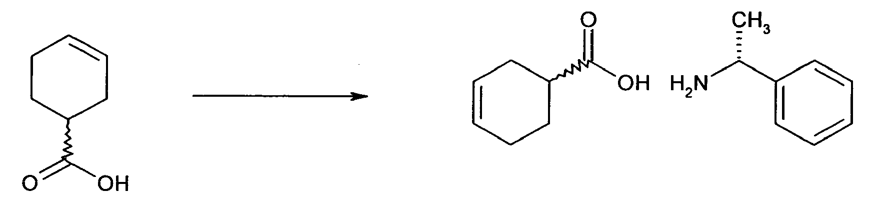

191.93 g of racemic cyclohex-3-ene-1-carboxylic acid were charged to a flask and 5 volumes of acetone were added. The mixture obtained was stirred, heated to 55 to 60°C and stirred for approximately 30 min. To the mixture obtained 1.0 equivalent of (*R*)-(+)-methylbenzylamine in 2 volumes of acetone were added dropwise over approximately 50 min. A clear, yellow solution was obtained and refluxed for 2 h. The solution obtained was cooled slowly to rt. Crystallisation started at approximately 50°C. Once at rt from the mixture obtained the precipitate obtained was filtered off, washed with acetone and dried in vacuo at 40°C overnight. A methylbenzylamine salt of cyclohex-3-ene-1-carboxylic acid as set out in the reaction scheme above was obtained.
Yield: 251.76 g (66.9% of theory); Optical Rotation: [α]_{D} (c=5.12, MeOH) = -7.7°

### B. Resolution

A portion of the salt from step A and 5 volumes of acetone were charged to a flask and heated to reflux. Further acetone was added in order to ensure complete dissolution. When the product had dissolved, the mixture obtained was stirred for a further 15 min under reflux and slowly cooled to rt. The precipitate obtained was filtered off and washed with acetone. A methylbenzylamine salt of cyclohex-3-ene-1-carboxylic acid wherein the S-isomer was enriched was obtained.
Yield: Weight (wet): 161.15 g; optical rotation: ²⁰[α]_{D} = -25.8° (c=5.16, MeOH)

The above procedure is repeated until required optical rotation (²⁰[α]_{D} >-40°) is achieved.

### C. Salt Release

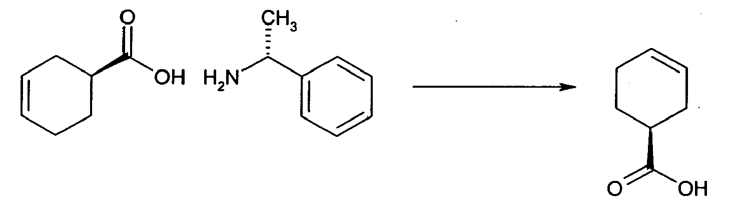

54.94 g of cyclohex-3-ene-1(*S*)-carboxylic acid (R)-(+)-methylbenzylamine salt and 18.2 volumes of H₂O were charged to a flask at 20-25°C and stirred. To the mixture obtained further 6.7 volumes of H₂O and 10 volumes of EtOAc were added. The mixture obtained was stirred for 15 min, the phases were separated and the organic layer was discarded. The aqueous layer was treated with 10 volumes of MTBE and 2.5 equivalents of 6M HCl were added slowly. The mixture obtained was stirred and two layers were formed, separated and the aqueous layer was extracted with MTBE. The organic layers obtained were combined and washed with aqueous, 30% NaCl solution. The organic phase obtained was dried over Na₂SO₄, filtered, and from the filtrate obtained solvent was removed in vacuo. Cyclohex-3-ene-1(*S*)-carboxylic acid in the form of a clear oil was obtained.
Yield: 26.13 g (93.3% of theory); Optical Rotation: [α]_{D} (c=4.66, MeOH) = -91.3°

Cyclohex-3-ene-1(*S*)-carboxylic acid may be obtained in analogy to the method disclosed in Schwartz, H. M.; et al. JACS 1978, 100, 5199-5203.

### 3. Curtius Rearrangement

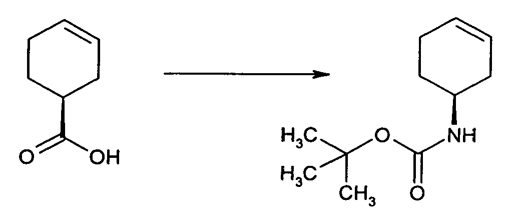

26.13 g of cyclohex-3-ene-1(*S*)-carboxylic acid and 14.9 volumes of toluene were charged to a flask at 20-25°C and stirred. To the mixture obtained 1.1 equivalents of NEt₃ were added dropwise, followed by 1.0 equivalents of DPPA. The mixture obtained was heated to 60°C and stirred for 15 min and heated further to reflux. The progress of the reaction was followed by ¹H NMR measurments until completion. The mixture obtained was cooled to 50°C over 40 min and 5 equivalents of tert-butanol were added dropwise over 10 min, followed by 0.04 equivalents of CuCl. The mixture obtained was heated to reflux. Progress of the reaction was followed by ¹H NMR measurments until completion. The mixture obtained was cooled and 5 volumes of aqueous, 30% NaHCO₃ solution were added. The mixture obtained was stirred for 15 min, filtered through celite in order to remove residual solid and the solid obtained was washed with toluene.The organic layers obtained were separated and the aqueous layer obtained was washed with toluene. All organic layers obtained were combined, washed with aqueous saturated NaCl solution, dried and solvent was removed in vacuo. *tert*-Butyl cyclohex-3-enyl-1(*S*)-carbamate was obtained in the form of a brown solid.
Crude Yield: 39.18 g; Optical Rotation: [α]_{D} (CHCl₃) = -16.7°

The crude product obtained was subjected to column chromatography (eluent: Cyclohexane/EtOAc 9: 1). The required fractions were identified, combined and concentrated in vacuo. *tert*-Butyl cyclohex-3-enyl-1(*S*)-carbamate in the form of a white solid was obtained.
Yield: 62.2% of theory; Optical Rotation: [α]_{D} (c=5.70, CHCl₃) = -18.3°
¹H NMR (CDCl₃, 200 MHz, ppm): δ 5.52-5.68 (m, 2H), 4.56 (s, broad, 1H), 3.75 (s, broad, 1H), 2.29-2.42 (m, 1H), 2.07-2.11 (m, 2H), 1.76-1.90 (m, 2H), 1.48-1.56 (m, 1H), 1.43 (s, 9H)

### Example 15

### tert-Butyl (1S,3S,6R)-(7-oxabicyclo[4.1.0]hept-3-yl)-carbamate

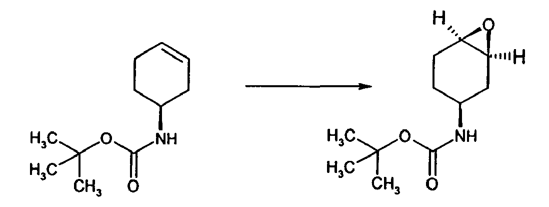

36.51 g of 70% mCPBA and 195 mL of CH₂Cl₂ were charged to a 1L flask and cooled using an ice bath. To the mixture obtained 24.34 g of *tert*-butyl cyclohex-3-enyl-1(*S*)-carbamate in 37 mL of CH₂Cl₂ were added dropwise over approximately 30 minutes ensuring a temperature below 30°C. 12 mL of further CH₂Cl₂ were added and the mixture obtained was heated to reflux (40°C) until the reaction was completed (¹H NMR control). The mixture obtained was cooled to 0-5°C and the solid precipitate was filtered off and washed with CH₂Cl₂. The filtrate obtained was washed with 10% sodium thiosulfate in order to remove peroxides, saturated aqueous NaHCO₃ solution in order to ensure a pH>7 (recorded pH=9) in the aqueous phase, and water. The filtrate obtained was concentrated, 122 mL of toluene were added and the mixture obtained was concentrated again. A further portion of 122 mL of toluene was added and the mixture obtained was concentrated to approximately 2 volumes. The mixture obtained comprising *tert*-butyl (1*S*,3*S*,6*R*)-(7-oxabicyclo[4.1.0]hept-3-yl)-carbamate was kept cool (refrigerator) until required in the next step. From the solution_*tert-*butyl (1*S*,3*S*,6*R*)-(7-oxabicyclo[4.1.0]hept-3-yl)-carbamate_was obtained in solid form upon concentration.
Yield: 17.27g
¹H NMR (200 MHz, DMSO-d₆, ppm) δ 6.63 (d, J=7Hz, 1H), 3.28 - 3.09 (m, 1H), 3.03 (s, broad, 2H), 2.15 - 1.96 (m, 2H), 1.85 - 1.67 (m, 2H), 1.59 - 1.27 (m, 11H)

### Example 16

### tert-Butyl (1R,3R,6S)-(7-oxabicyclo[4.1.0]hept-3-yl)-carbamate

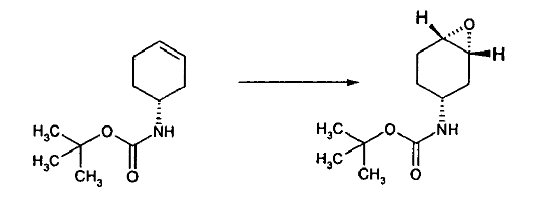

4500 g of mCPBA (70%) and 24 L of CH₂Cl₂ were charged to a vessel and cooled to 15°C. 3000 g of *tert*-butyl cyclohex-3-enyl-1(*R*)-carbamate in 4.5 L of of CH₂Cl₂ were added dropwise over approximately 30 min maintaining the temperature at 15 to 25°C. To the mixture obtained 1.5 L of CH₂Cl₂ were added and the mixture obtained was stirred at 20 to 25°C for 1 h and heated to reflux (40°C) for 2 h. Upon completion of the reaction (¹H NMR control), the mixture was cooled to -5 to 0°C, stirred for 30 min and the solid precipitate was filtered off and washed with CH₂Cl₂. The resultant filtrate was washed with 10% aqueous sodium thiosulfate solution in order to remove peroxides, saturated aqueous NHCO₃ solution until a pH>7 was achieved in the aqueous phase, and water. The organic phase obtained was concentrated to minimal volume and 15 L of toluene were added and the mixture was concentrated again to minimal volume. A further portion of 15 L of toluene was added and the mixture obtained was concentrated to approximately 2 volumes to produce *tert*-butyl (1*R*,3*R*,6*S*)-(7-oxabicyclo[4.1.0]hept-3-yl)-carbamate in the form of a solution in toluene. From the solution *tert*-butyl (1*R*,3*R*,6*S*)-(7-oxabicyclo[4.1.0]hept-3-yl)-carbamate was obtained in solid form upon concentration.
Crude Yield: 2.63 kg
Pure Yield: 2.05 kg
¹H NMR (200 MHz, CDCl₃, ppm) δ 4.85 (d, J=7Hz, 1H), 3.6 - 3.54 (m, 1H), 3.10 (s, broad, 2H), 2.23 - 1.99 (m, 2H), 1.92 - 1.67 (m, 2H), 1.54 -1.27 (m, 11H)

### Example 17

### {(1S,2S,4S)-4-[(tert-Butoxycarbonyl)-amino]-2-hydroxy-cyclohexyl}-benzene-carbothioate

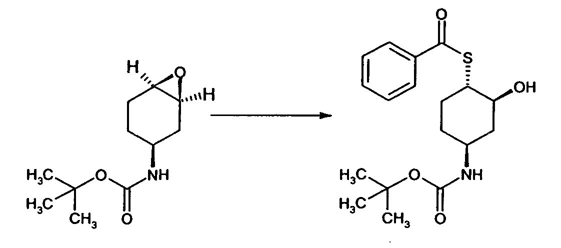

17.27 g of *tert*-butyl (1*S*,3*S*,6R)-(7-oxabicyclo[4.1.0]hept-3-yl)-carbamate and 52 mL of toluene were charged to a 1L flask and stirred at 15-25°C. To the mixture obtained 13 mL of thiobenzoic acid (melted) were added dropwise. The temperature was kept below 30°C. Further 16 mL of toluene and 0.7g of tetrabutylammonium chloride monohydrate in one portion were added, external temperature control was stopped, and the mixture obtained was subjected to exotherm reaction. The mixture obtained was heated to 40-45°C. Upon completion of the reaction (TLC control), the mixture obtained was cooled to rt and washed with 5% aqueous NaHCO₃ solution followed by H₂O. The organic layer obtained was concentrated *in vacuo* to minimum volume. 86 mL of toluene were added and the mixture obtained was again concentrated to minimum volume. That process was repeated.
To the crude concentration residue obtained 17 mL of toluene were added under stirring, at which point a solid precipitated. 17 mL of heptane were added dropwise and the mixture was stirred at rt overnight. After stirring, the solid obtained was filtered off, dried and washed with 9 mL of toluene-heptane (1:1), followed by a slurry wash with 17 mL of toluene-heptane 1:1, followed by a further slurry wash with 35 mL of toluene-heptane 1:1. That procedure reduced the amount of regioisomer practically to undetectable amounts (determined by ¹H NMR).
The material which remained as crystalline solid was dried *in vacuo* at <40°C to give {(1*S*,2*S*,4*S*)-4-[(*tert*-Butoxycarbonyl)-amino]-2-hydroxy-cyclohexyl}-benzene-carbothioate in the form of a white solid.
Crude Yield: 9.41 g
Optical Rotation: [α]_{D} (c=3.73, CHCl₃) = +24.3°
¹H NMR (200 MHz, DMSO-d₆, ppm) δ 7.92 - 7.87 (m, 2H), 7.71 - 7.63 (m, 1H), 7.58 - 7.50 (m, 2H), 6.85 (d, J=8Hz, 1H), 5.11 (d, J=5.6Hz, 1H), 3.49 - 3.25 (m, 3H), 2.12 -1.95 (m, 2H), 1.79 - 1.69 (m, 1H), 1.54 - 1.14 (m, 12H)

### Example 18

### {(1R,2R,4R)-4-[(tert-Butoxycarbonyl)-amino]-2-hydroxy-cyclohexyl}-benzene-carbothioate

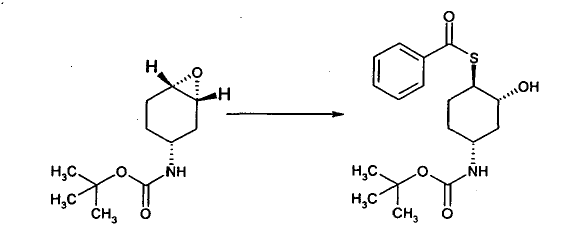

2050 g of *tert*-butyl (1*R*,3*R*,6*S*)-(7-oxabicyclo[4.1.0]hept-3-yl)-carbamate and 6.15 L of toluene were charged to a vessel and stirred at 15-25°C. To the mixture obtained 1.52 L of thiobenzoic acid (10%) were added dropwise. The temperature was kept below 30°C. Further 1.85 L of toluene and 85.5 g of tetrabutylammonium chloride monohydrate in one portion were added, external temperature control was stopped, and the mixture obtained was subjected to exotherm reaction. The mixture obtained was heated to 40-45°C and stirred for 4 h. Upon completion of the reaction (TLC and ¹H NMR-control), the mixture obtained was cooled to 15 to 20°C and washed with 5% aqueous NaHCO₃ solution followed by H₂O. The organic layer obtained was concentrated *in vacuo* to minimum volume. 10.25 L of toluene were added and the mixture obtained was again concentrated to minimum volume. That process was repeated.
To the crude concentration residue obtained 0.5 volumes of toluene were added under stirring and the mixture obtained was stirred at 15 to 25°C for 30 min. To the mixture obtained 0.5 volumes of heptane were added the mixture was stirred at 15 to 25°C for 1 h. After stirring, the solid obtained was filtered off, dried and washed with toluene-heptane (1:1), followed by a slurry wash toluene-heptane 1:1, followed by a further wash with toluene-heptane 1:1. That procedure reduced the amount of regioisomer practically to undetectable amounts (by ¹H NMR). The material which remained as solid was dried *in vacuo* at <40°C to give {(1*R*,2*R*,4*R*)-4-[(*tert*-Butoxycarbonyl)-amino]-2-hydroxycyclohexyl}-benzene-carbothioate in the form of a white solid.
Overall Yield: 3950 g
¹H NMR (200 MHz, DMSO-d₆, ppm) δ 7.92 - 7.87 (m, 2H), 7.71 - 7.63 (m, 1H), 7.58 - 7.,49 (m, 2H), 6.85 (d, J=8Hz, 1H), 5.11 (d, J=5.6Hz, 1H), 3.49 - 3.25 (m, 3H), 2.12 -1.95 (m, 2H), 1.79 - 1.69 (m, 1H), 1.54 -1.14 (m, 12H)

## Claims

1. A process for the preparation of a compound of formula 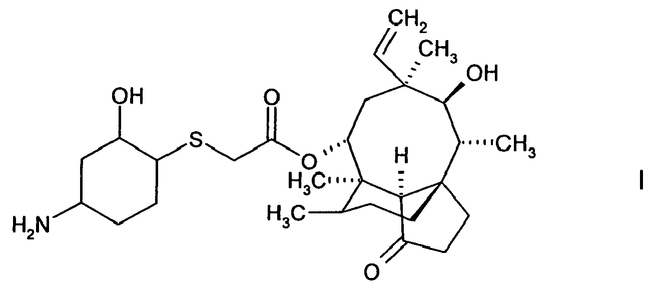 in the form of a single stereoisomer,
optionally wherein a compound of formula I in the form of a single stereoisomer is a compound of formula 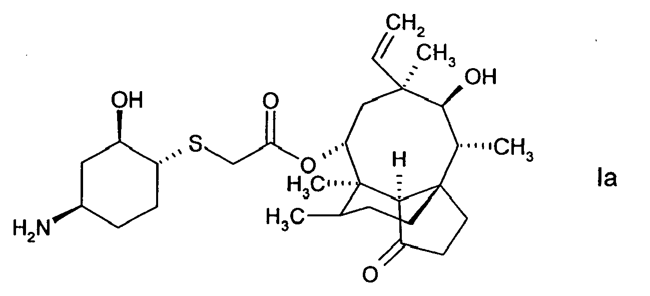 or a compound of formula 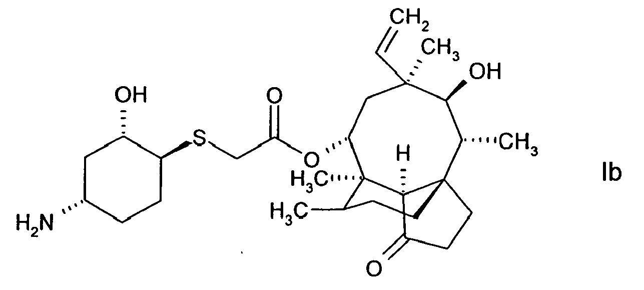 comprising deprotecting the amine group in an N-protected amino-hydroxy-cyclohexylsulfanyl-acetyl-mutilin of formula 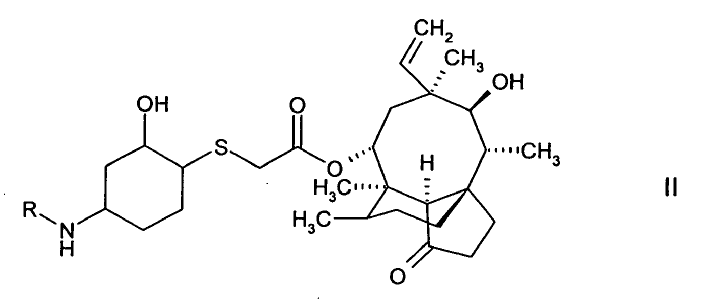 wherein R is an amine protecting group,
in the form of a single stereoisomer,
optionally wherein a compound of formula II in the form of a single stereoisomer is a compound of formula 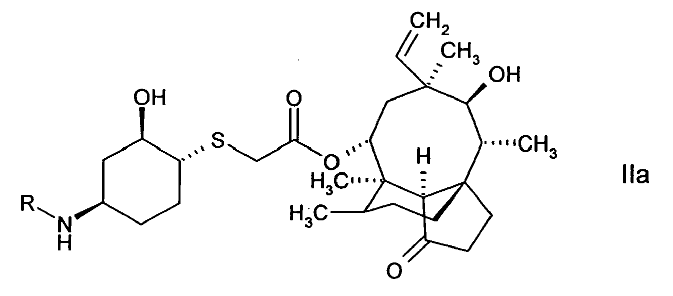 or a compound of formula 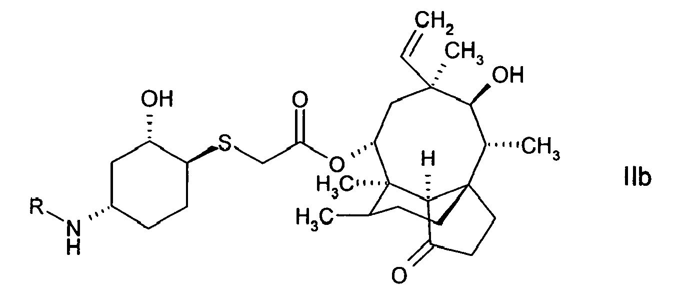 wherein R is as defined above;
and isolating a compound of formula I, optionally a compound of formula Ia or Ib, in the form of a single stereoisomer obtained from the reaction mixture.

2. A compound of formula I, optionally of formula Ia, as defined in claim 1, in the form of a single stereoisomer according to claim 1, in crystalline form.

3. A process or a compound according to any one of claims 1 or 2, wherein a compound of formula I is a compound of formula

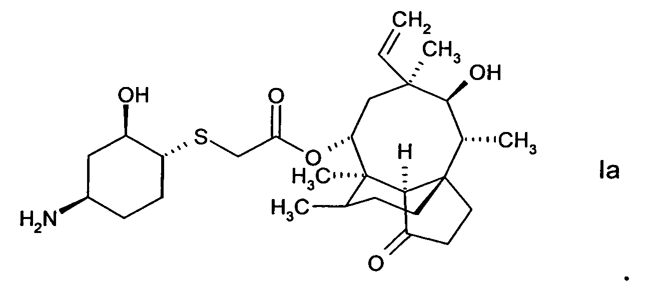

4. A compound according to claim 3, which is 14-*O*-{[(1*R*,2*R*,4*R*)-4-amino-2-hydroxy-cyclohexylsulfanyl]-acetyl}-mutilin in crystalline Form 1.

5. A compound according to claim 3, which is 14-*O*-{[(1*R*,2*R*,4*R*)-4-amino-2-hydroxy-cyclohexylsulfanyl]-acetyl}-mutilin in crystalline Form 2.

6. A compound of formula I as defined in one of claims 1 to 5, in the form of a single stereoisomer according to claim 1 in the form of a crystalline salt.

7. A salt according to claim 6, which salt is an acetate, lactate or maleate.

8. A compound according to any one of claims 6 or 7, which is selected from the group consisting of
14-*O*-{[(1*R*,2*R*,4*R*)-4-amino-2-hydroxy-cyclohexylsulfanyl]-acetyl}-mutilin acetate in crystalline Form A;
14-*O*-{[(1*R*,2*R*,4*R*)-4-amino-2-hydroxy-cyclohexylsulfanyl]-acetyl}-mutilin acetate in crystalline Form B
14-*O*-{[(1*R*,2*R*,4*R*)-4-amino-2-hydroxy-cyclohexylsulfanyl]-acetyl}-mutilin L-lactate in crystalline Form 1, and
14-*O*-{[(1*R*,2*R*,4*R*)-4-amino-2-hydroxy-cyclohexylsulfanyl]-acetyl}-mutilin maleate in crystalline Form 1.

9. A process according to claim 1 or claim 3, wherein a compound of formula II in the form of a single stereoisomer,
which compound of formula II in the form of a single stereoisomer optionally is a compound of formula IIa or IIb as defined in claim 1,
is obtained by coupling a compound of formula 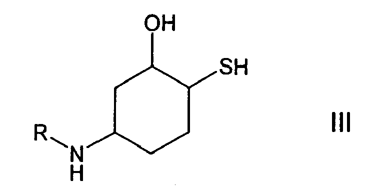 wherein R is as defined in claim 1, in the form of a single stereoisomer, which compound of formula III optionally is a compound of formula 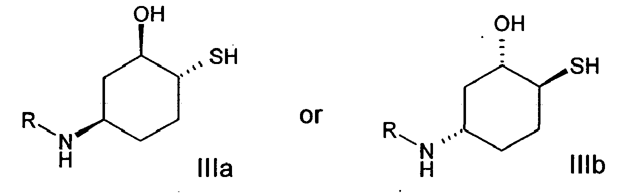 wherein R is as defined in claim 1,
with an activated 14-O-AKT-acetyl-mutilin, wherein AKT is an activating group, optionally mesyl, besyl, tosyl, optionally 14-O-AKT-acetyl-mutilin is a compound of formula 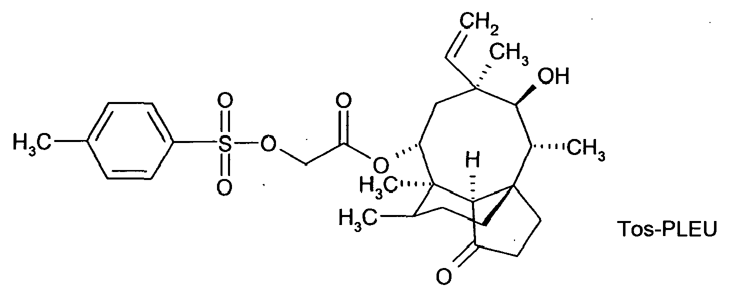 and isolating a compound of formula II, optionally a compound of formula IIa or IIb as defined in claim 1, obtained from the reaction mixture.

10. A process according to claim 9, wherein a compound of formula III, wherein R is as defined in claim 9, in the form of a single stereoisomer,
which compound of formula III in the form of a single stereoisomer optionally is a compound of formula IIIa or IIIb as defined in claim 9,
is obtained by deprotecting the thiol function in a compound of formula 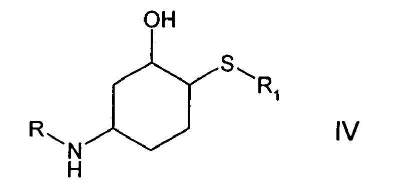 in the form of a single stereoisomer, wherein a compound of formula IV in the form of a single stereoisomer optionally is a compound of formula 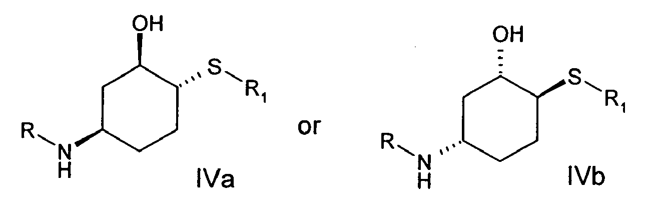 wherein R is as defined in claim 9 and R₁ is a thiol protecting group,
and isolating a compound of formula III in the form of a single stereoisomer, optionally a compound of formula IIIa or IIIb, obtained from the reaction mixture.

11. A compound of formula II in the form of a single stereoisomer as defined in claim 9, and/or a compound of formula III in the form of a single stereoisomer as defined in claim 9, for use as an intermediate in a process for the production of a compound of formula I in the form of a single stereoisomer, as defined in claim 1.

12. Pharmaceutical compositions comprising crystalline 14-O-{[(-4-amino-2-hydroxycyclohexyl)sulfanyl]acetyl}mutilin, or comprising an, optionally crystalline, acetate, lactate, or maleate of 14-O-{[(-4-amino-2-hydroxycyclohexyl)sulfanyl]acetyl}mutilin.

13. A compound of formula III, optionally in the form of a single stereoisomer, optionally in the form of a compound of formula IIIa or IIIb as defined in claim 9.

14. A compound of formula IIa or IIb, as defined in claim 1.
